# EUROPEAN PATENT SPECIFICATION

(11) **EP 3 307 265 B1**
(45) Date of publication and mention of the grant of the patent: **13.12.2023**
(21) Application number: 16806447.5
(22) Date of filing: 10.06.2016
(51) Int. Cl.: A61K 31/343, A61K 31/33, A61K 31/381, A61K 31/404, A61K 39/395, A61P 35/00

(54) **PHARMACEUTICAL COMBINATION AND USES THEREOF**
PHARMAZEUTISCHE KOMBINATION UND VERWENDUNGEN DAVON
ASSOCIATION PHARMACEUTIQUE ET UTILISATIONS DE CETTE ASSOCIATION

(30) Priority: 11.06.2015 AU 2015902260
(43) Date of publication of application: 18.04.2018
(73) Proprietor: Bionomics Limited, Eastwood SA 5063 (AU)
(72) Inventor: KREMMIDIOTIS, Gabriel, Flagstaff Hill, South Australia 5159 (AU); LAVRANOS, Tina, Colonel Light Gardens, South Australia 5041 (AU); INGLIS, Daniel, Clarence Park, South Australia 5034 (AU)
(74) Representative: Gevers Patents
(86) International application number: PCT/AU2016/050478
(87) International publication number: WO 2016/197204

(56) References cited:
- EP-A1- 2 191 841
- WO-A1-2007/087684
- WO-A1-2011/022781
- WO-A1-2016/130839
- WO-A1-2016/141209
- WO-A2-2007/113648
- WO-A2-2007/123737
- WO-A2-2008/089070
- WO-A2-2010/014784
- WO-A2-2015/191602
- US-A1- 2011 130 367
- TINA C. LAVRANOS ET AL: "Abstract 4039: Harnessing the tumor adaptive response to hypoxia to identify novel combinations of the vascular disrupting agent BNC105 with targeted therapeutics.", CANCER RESEARCH, vol. 73, no. 8 Supplement, 15 April 2013 (2013-04-15), pages 4039-4039, XP055212007, ISSN: 0008-5472, DOI: 10.1158/1538-7445.AM2013-4039
- G. KREMMIDIOTIS ET AL: "BNC105: A Novel Tubulin Polymerization Inhibitor That Selectively Disrupts Tumor Vasculature and Displays Single-Agent Antitumor Efficacy", MOLECULAR CANCER THERAPEUTICS, vol. 9, no. 6, 1 June 2010 (2010-06-01), pages 1562-1573, XP055239369, US ISSN: 1535-7163, DOI: 10.1158/1535-7163.MCT-09-0815
- SHEETAL BODHANKAR ET AL: "Targeting immune co-stimulatory effects of PD-L1 and PD-L2 might represent an effective therapeutic strategy in stroke", FRONTIERS IN CELLULAR NEUROSCIENCE, vol. 8, 11 August 2014 (2014-08-11), XP055392284, DOI: 10.3389/fncel.2014.00228
- K. S. PEGGS ET AL: "Blockade of CTLA-4 on both effector and regulatory T cell compartments contributes to the antitumor activity of anti-CTLA-4 antibodies", SCIENCE, vol. 322, no. 5899, 3 August 2009 (2009-08-03), pages 271-1725, XP055100399, ISSN: 0036-8075, DOI: 10.1084/jem.20082492
- Tina C Lavranos ET AL: "The tubulin-targeting agent BNC105 potentiates the efficacy of immune checkpoint inhibitors in preclinical models of colorectal cancer", , 12 November 2015 (2015-11-12), XP055446893, Retrieved from the Internet: URL:http://www.bionomics.com.au/upload/res earch-development/pipeline/oncology/BNC105 _-Immuno_MTCT2015.pdf [retrieved on 2018-02-01]
- Anonymous: "New BNC105 Clinical Trial In Combination With Keytruda", , 20 February 2017 (2017-02-20), XP055446834, Retrieved from the Internet: URL:https://www.clinicalleader.com/doc/new -bnc-clinical-trial-in-combination-with-ke ytruda-0001 [retrieved on 2018-02-01]
- Daniel J Inglis ET AL: "Abstract 4982: BNC105 induces tumor micro-environment changes which enhance the efficacy of checkpoint inhibitor therapy in preclinical models | Cancer Research", , 1 July 2016 (2016-07-01), XP055446693, Retrieved from the Internet: URL:http://cancerres.aacrjournals.org/cont ent/76/14_Supplement/4982 [retrieved on 2018-02-01]
- INGLIS, D.J. ET AL.: 'The vascular disrupting agent BNC105 potentiates the efficacy of VEGF and mTOR inhibitors in renal and breast cancer' CANCER BIOLOGY & THERAPY vol. 15, no. 11, 2014, pages 1552 - 1560, XP009185993
- MULLER, P. ET AL.: 'Microtubule-Depolymerizing Agents Used in Antibody-Drug Conjugates Induce Antitumor Immunity by Stimulation of Dendritic Cells' CANCER IMMUNOL RES vol. 2, no. 8, 2014, pages 741 - 755, XP055334020
- ASX ANNOUNCEMENT: 'Bionomics Files Patent for Use of BNC105 in Combination with PD- 1 and CTLA-4 Immuno-Oncology Antibodies', [Online] 15 June 2015, pages 1 - 2, XP003034337 Retrieved from the Internet: <URL:HTTP://WWW.ASX.COM.AU/ASXPDF/20150615/ PDF/42Z61SNX70C1PY.PDF> [retrieved on 2016-09-14]
- LAVRANOS, T.C. ET AL.: 'The tubulin-targeting agent BNC105 potentiates the efficacy of immune checkpoint inhibitors in preclinical models of colorectal cancer' MOLECULAR CANCER THERAPEUTICS vol. 14, no. 12, 2015, XP009502776
- Anonymous: "BNC105 | Cas# 945771-74-4 - GlpBio", , 16 December 2020 (2020-12-16), XP055760474, Retrieved from the Internet: URL:https://www.glpbio.com/bnc105.html [retrieved on 2020-12-16]
- Anonymous: "BNC105 | Microtubule/Tubulin Inhibitor | MedChemExpress", , 16 December 2020 (2020-12-16), XP055760475, Retrieved from the Internet: URL:https://www.medchemexpress.com/BNC105. html [retrieved on 2020-12-16]

## Description

### FIELD

The field of the invention relates to a combination of a vascular disrupting agent and an immunotherapeutic agent for use in the treatment of cancer. The field of the invention further relates to a combination of a vascular disrupting agent and an immunotherapeutic agent for use in treating cancer by administering the combination to a patient in need thereof.

### BACKGROUND

An intact and functioning vascular network is critical for the development, growth, and survival of most solid tumors. Without proper blood flow, tumors are unable to grow more than a few millimeters and dormancy occurs. Although the architecture of normal tissue vasculature is refined, with organized and regular structure, vasculature in the tumor is comparatively formless, with random connections that result in chaotic blood flow. Blood vessels within tumors contain an abnormal endothelial cell physiology that provides a potential focus for affecting vascular growth and viability. Therefore, the tumor vasculature has become an attractive target for antineoplastic therapies, and several novel agents that target components of the tumor vasculature are currently in clinical development.

Vascular disrupting agents (VDAs) are a class of drugs that target tumor vasculature and induce a rapid collapse and regression of tumor vessels, with a consequent deprivation of blood and oxygen leading to necrosis of the tumor. Unlike anti-angiogenic drugs, VDAs occlude the pre-existing blood vessels of tumors to cause cell death from ischemia and extensive necrosis.

Although vascular disrupting agents have demonstrated signs of preclinical and clinical activity in different tumor types, particularly in combination with cancer chemotherapeutics, a number of trials were unable to meet primary endpoints in randomized patient populations. As a consequence, investigations are being conducted to define patient subpopulations that would most likely benefit from treatment with a vascular disrupting agent. There is also some evidence that the use of concomitant medicines in clinical trials may compromise the antitumor action of certain vascular disrupting agents. Accordingly, there remains a need for further improvement to methods of treating cancer with a vascular disrupting agent.

US 2011/130367 describes a pharmaceutical combination for treating a proliferative disease such as cancers (paragraphs [0005] and [0040]). The pharmaceutical combination described in this document comprises: (a) BNC105, and; (b) at least one other anti-proliferative agent selected from alkylating agents, antitumor antibiotics, antimetabolites, natural alkaloids and inhibitors of protein tyrosine kinases and/or serine/threonine kinases (paragraph [0005]). The function of anti-proliferative agents is to suppress, inhibit or prevent the cell proliferation from happening. US 2011/130367 does not describe the presence of an anti-immune checkpoint inhibitor antibody in the pharmaceutical combination. The compound used together with BNC105 in US 2011/130367 is for inhibiting or suppressing the cell proliferation, which has different functions from an anti-immune checkpoint inhibitor antibody.

Furthermore, neither of Tina C. Lavranos, *et al.,* (15 April, 2013) or Inglis, D. J., *et al.,* (2014) describes or suggests the combination of BCN105 and an anti-immune checkpoint inhibitor antibody. Tina C. Lavranos, *et al.,* (15 April, 2013) discloses the combination of BNC105 and the proteasome inhibitor Bortezomib in the treatment of RENCA tumors and the combination of BNC105 and the mTOR inhibitor Rapamycin used to inhibit mTOR signalling for use in the treatment of mice bearing A498 tumor. Inglis, D. J., *et al.,* (2014) describes that BNC105 potentiates the efficacy of VEGF and mTOR inhibitors in renal and breast cancer.

### SUMMARY

The invention is as defined in claims 1 to 5.

The present inventors have determined that a combination of a vascular disrupting agent and an immunotherapeutic agent increases the efficacy of treating cancer when compared to treatment with either the vascular disrupting agent or the immunotherapeutic agent alone.

Accordingly, a first aspect provides:
(i) a vascular disrupting agent, and
(ii) an immunotherapeutic agent,
for use in the treatment of cancer as claimed in claim 1.

A second aspect described herein but not part of the claimed invention provides a method for the treatment of cancer, the method comprising administering to a cancer patient a vascular disrupting agent and an immunotherapeutic agent.

A third aspect described herein but not part of the claimed invention provides a method for the treatment of cancer, the method comprising administering an immunotherapeutic agent to a cancer patient undergoing treatment with a vascular disrupting agent.

A fourth aspect described herein but not part of the claimed invention provides a method for the treatment of cancer, the method comprising administering a vascular disrupting agent to a cancer patient undergoing treatment with an immunotherapeutic agent.

One embodiment not within the scope of the claimed invention describes a pharmaceutical composition comprising a vascular disrupting agent and an immunotherapeutic agent.

One embodiment not within the scope of the claimed invention describes use of a vascular disrupting agent and an immunotherapeutic agent in the manufacture of a medicament for the treatment of cancer.

One embodiment of not within the scope of the claimed invention describes use of an immunotherapeutic agent in the manufacture of a medicament for the treatment of cancer in a patient, wherein the patient is undergoing treatment with a vascular disrupting agent.

One embodiment of not within the scope of the claimed invention describes use of a vascular disrupting agent in the manufacture of a medicament for the treatment of cancer in a patient, wherein the patient is undergoing treatment with an immunotherapeutic agent.

In an embodiment as claimed in claim 1, there is provided a vascular disrupting agent and an immunotherapeutic agent for use in the treatment of cancer as claimed in claim 1.

In an embodiment as claimed in claim 2, there is provided an immunotherapeutic agent for use in the treatment of cancer in a cancer patient undergoing treatement a vascular disrupting agent.

In an embodiment as claimed in claim 3, there is provided a vascular disrupting agent for use in the treatment of cancer in a cancer patient undergoing treatment with an immunotherapeutic agent.

In one embodiment not part of the claimed invention, the pharmaceutical combination for the treatment of cancer comprises:
(i) a first pharmaceutical composition comprising a vascular disrupting agent, and
(ii) a second pharmaceutical composition comprising an immunotherapeutic.

In another embodiment not part of the claimed invention, the pharmaceutical combination for the treatment of cancer comprises:
a single pharmaceutical composition comprising a vascular disrupting agent, an immunotherapeutic agent, and a pharmaceutical carrier or excipient.

In one embodiment described herein, the vascular disrupting agent is conjugated to the immunotherapeutic agent.

The vascular disrupting agent is a tubulin polymerisation inhibitor.

In one particular embodiment described herein but not part of the claimed invention, the tubulin polymerisation inhibitor is selected from ABT-751, MPC-6827, AEZS-112, CYT997, MN-029, EPC2407, ZIO-301, vinflunine, vinblastine, vincristine, CA4, Oxi4503, AVE8062, eribulin mesylate, dolastatin, tasidotin, 2-methoxyestradiol, E7974 and/or NPI-2358.

In one embodiment described herein but not part of the claimed invention, the tubulin polymerisation inhibitor is a compound of formula (I) or a salt, solvate or prodrug thereof 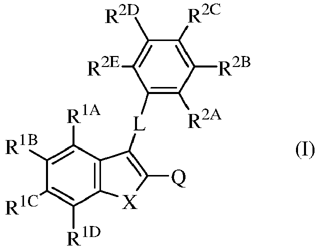 wherein;
X represents O, S, SO, SO₂, Se, SeO, SeO₂ or NR where R is selected from H, O, optionally substituted acyl, optionally substituted alkenyl, optionally substituted alkyl, optionally substituted aryl, optionally substituted cycloalkenyl, optionally substituted cycloalkyl, optionally substituted heteroaryl, optionally substituted heterocyclyl, and optionally substituted sulfonyl;
R^{1A} and R^{1B} each independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacyloxy, optionally substituted oxyacylimino, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or R^{1A} and R^{1B} together form an optionally substituted aryl, optionally substituted heterocyclyl, optionally substituted heteroaryl, optionally substituted cycloalkyl, or optionally substituted cycloalkenyl;
R^{1C} represents C₁₋₃ alkoxy, C₁₋₃ alkylthio, C₁₋₃ alkylamino, or C₁₋₃ dialkylamino;
R^{1D} represents hydroxy or amino;
L represents C=O, O, S, SO, SO₂, Se, SeO, SeO₂, C=NZ', or NR' where Z' is H, optionally substituted alkyl, optionally substituted aryl or optionally substituted amino; and where R' is selected from H, O, optionally substituted acyl, optionally substituted alkenyl, optionally substituted alkyl, optionally substituted aryl, optionally substituted cycloalkenyl, optionally substituted cycloalkyl, optionally substituted heteroaryl, optionally substituted heterocyclyl, or optionally substituted sulfonyl;
R^{2A}-R^{2E} each independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacylimino, optionally substituted oxyacyloxy, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or optionally substituted thioacyloxy; or any of R^{2A} and R^{2B}, R^{2B} and R^{2C}, R^{2C} and R^{2D}, and R^{2D} and R^{2E}, together form an optionally substituted aryl, optionally substituted heterocyclyl, optionally substituted heteroaryl, optionally substituted cycloalkyl, or optionally substituted cycloalkenyl; and
Q represents H, CN, halogen, trialkylsilyl, optionally substituted alkyl, optionally substituted alkenyl, optionally substituted alkynyl, optionally substituted acyl, optionally substituted oxyacyl, optionally substituted acylamino, optionally substituted aminoacylamino, OR", SR" or NR"R", where each R" independently represents, H, optionally substituted alkyl, optionally substituted alkenyl, optionally substituted alkynyl, optionally substituted aryl, optionally substituted heteroaryl, optionally substituted heterocyclyl, optionally substituted acyl and optionally substituted oxyacyl, or NR‴NR‴, where each R‴ independently represents H, optionally substituted alkyl, optionally substituted alkenyl, optionally substituted alkynyl, optionally substituted aryl and optionally substituted heteroaryl.

In one embodiment described herein but not part of the claimed invention, the compound of formula (I) is a prodrug selected from an ester, an acetate, a phosphate ester or an amide prodrug. In another embodiment, the compound of formula (I) is a phosphate prodrug. In a particular embodiment, R^{1D} is hydroxy and the prodrug is a phosphate ester of the hydroxy group. Preferably, the phosphate ester is a disodium phosphate ester.

In yet another embodimentdescribed herein but not part of the claimed invention, the tubulin polymerisation inhibitor is a compound of formula (III) or a salt, solvate or prodrug thereof

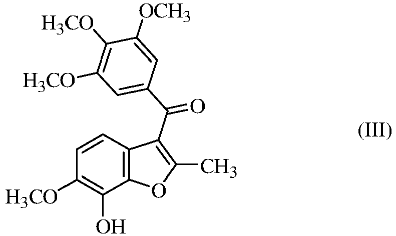

In one particular embodiment falling within the scope of the claimed invention, the tubulin polymerisation inhibitor is selected from 2-methyl-7-hydroxy-3-(3,4,5-trimethoxybenzoyl)-6-methoxybenzofuran (BNC105) and disodium [6-methoxy-2-methyl-3-(3,4,5-trimethoxybenzoyl)-1-benzofuran-7-yl] phosphate (BNC105P).

In one embodiment falling within the scope of the claimed invention, the immunotherapeutic agent is an immune checkpoint inhibitor.

In one embodiment falling within the scope of the claimed invention, the immune checkpoint inhibitor is an inhibitor of an immune checkpoint protein selected from Programmed Death-Ligand 1 (PD-L1), CTLA-4. In an embodiment disclosed but not falling within the scope of the claimed invention, the immune checkpoint inhibitor is an inhibitor of an immune checkpoint protein selected from PD-L2, LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, GITR, HAVCR2, HVEM, IDO1, IDO2, ICOS (inducible T cell costimulator), KIR, LAIR1, LIGHT, MARCO (macrophage receptor with collageneous structure), PS (phosphatidylserine), OX-40, SLAM, TIGHT, VISTA, VTCN1, or any combination thereof.

In one particular embodiment falling within the scope of the claimed invention, the immune checkpoint inhibitor is an inhibitor of PD-L1, PD-1, or CTLA-4.

In one embodimentdescribed herein but not part of the claimed invention, the pharmaceutical combination further comprises an additional immunotherapeutic agent, or the method comprises administering a further immunotherapeutic agent.

In one embodiment described herein but not part of the claimed invention, the additional immunotherapeutic agent is an immune checkpoint inhibitor.

In one particular embodiment described herein but not part of the claimed invention, the pharmaceutical combination comprises an anti-PD-1 antibody and an anti-CTLA-4 antibody, or the method comprises the administration of an anti-PD-1 antibody and an anti-CTLA-4 antibody.

In one embodiment falling within the scope of the claimed invention, the immune checkpoint inhibitor is an anti-immune-checkpoint inhibitor antibody. In one particular embodiment falling within the scope of the claimed invention, the immune checkpoint inhibitor is ipilimumab. In yet another embodiment falling within the scope of the claimed invention, the immune checkpoint inhibitor is nivolumab.

In one embodiment, the cancer is a solid tumor. For example, in one embodiment falling within the scope of the claimed invention, the cancer is selected from bladder cancer, breast cancer, colon cancer, gastroenterological cancer, kidney cancer, lung cancer, ovarian cancer, pancreatic cancer, prostate cancer, proximal or distal bile duct cancer, or melanoma.

In one particular embodiment falling within the scope of the claimed invention, the cancer is colon cancer.

As understood in the art, a combination therapy may involve the administration of multiple pharmaceutical agents separately for the treatment of a disease, or alternatively, may involve the administration of multiple drugs as a combination formulation, i.e., a formulation containing multiple pharmaceutical active ingredients. In addition, where the drugs in a combination therapy are provided as separate formulations, the drugs may be administered concurrently or sequentially. Thus, in one embodiment of the pharmaceutical combination, the method or the use as described herein, the vascular disrupting agent and the immunotherapeutic agent are administered simultaneously, sequentially or separately.

In another embodiment described hereinbut not falling within the scope of the claimed invention, the vascular disrupting agent and the immunotherapeutic agents are co-formulated in a single composition.

In one embodiment not part of the invention, the medicament comprises:
(a) the vascular disrupting agent, wherein the medicament is for administration in combination with the immunotherapeutic agent; or
(c) the immunotherapeutic agent, wherein the medicament is for administration in combination with the vascular disrupting agent.

In one embodiment described herein but not falling within the scope of the clamed invention, BNC105P is administered at a dosage of about 8 mg/m² to about 16 mg/m². In one particular embodiment described herein but not falling within the scope of the claimed invention, BNC105P is administered at a dosage of 16 mg/m².

In yet another embodiment described herein but not within the scope of the claimed invention, the method comprises administering a further therapeutic agent and/or tumor irradiation to the patient.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention as claimed in claims 1 to 5, is hereinafter described by way of the following non-limiting Examples and with reference to the accompanying figures.

### BRIEF DESCRIPTION OF THE FIGURES

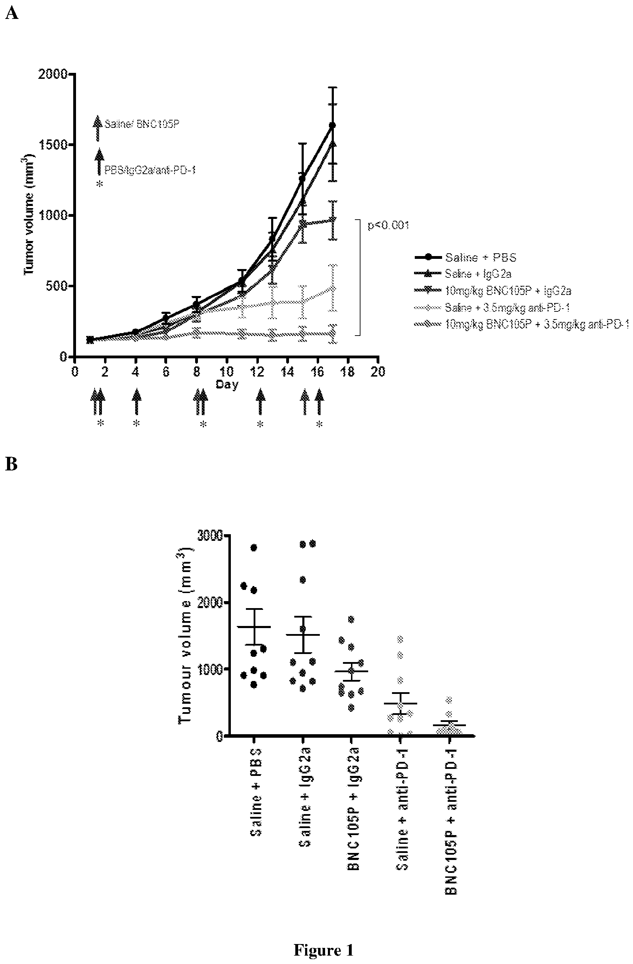

**Figure 1**. Evaluation of combining BNC105P with anti-PD1 in the MC38 colorectal cancer model. A) Line graph showing the tumor growth over the treatment period with tumor growth inhibition seen as early as Day 8 in the combination groups compared to the control group (p<0.05). B) Dot plot showing individual animal tumor volume in each of the treatment groups on Day 17. A 40% TGI was observed with BNC105P treated tumors, 74%TGI in anti-PD-1 treated tumors and 97% TGI in tumors treated with the BNC105P+anti-PD1 combination.

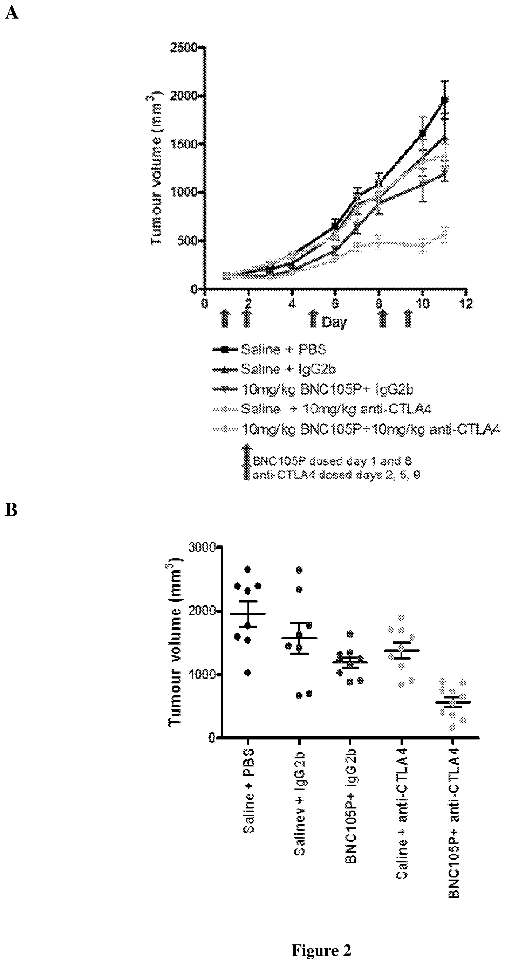

**Figure 2**. Evaluation of combining BNC105P with anti-CTLA4 in the CT26 colorectal cancer model. A) Line graph showing significant tumor growth inhibition over the treatment period in the combination group compared to the control groups (p<0.001). B) Dot plot showing individual animal tumor volume in each of the treatment groups on Day 11. A 27% tumor growth inhibition was observed in BNC105P treated animals, 14% tumor growth inhibition in anti-CTLA4 treated animals and 74% tumor growth inhibition in animals treated with the BNC105+anti-CTLA4 combination.

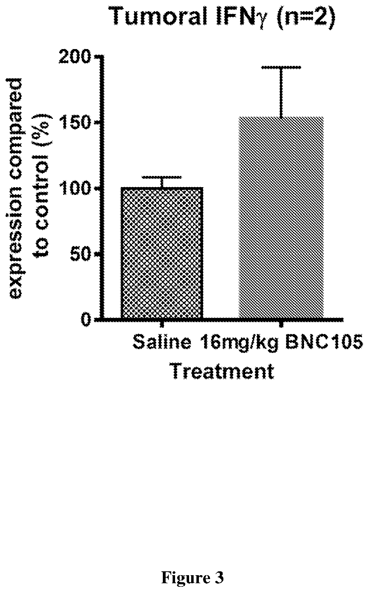

**Figure 3****.** Tumoral levels of IFNγ in animals administered with saline or 16 mg/kg BNC105.

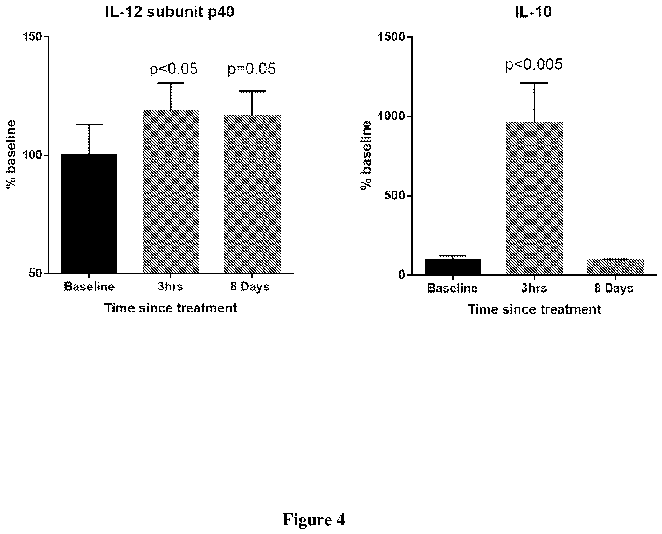

**Figure 4****.** Changes in levels of IL-12 p40 and IL-10 following administration with BNC105. Phase II Mesothelioma trial BNC105 (16mg/m2) number of patients =19. Blood draws were pre-specified and optional. Patients receiving BNC105 alone received blood draws prior to BNC105 administration and 3 hours following administration. Plasma samples were used to determine exploratory analytes using Multi-Analyte Profile (MAP) technology (Myriad RBM). Graph showing % change from baseline. Percent change was calculated as analyte plasma concentration (post - baseline) /baseline *100. Mean ± SEM shown on graph.

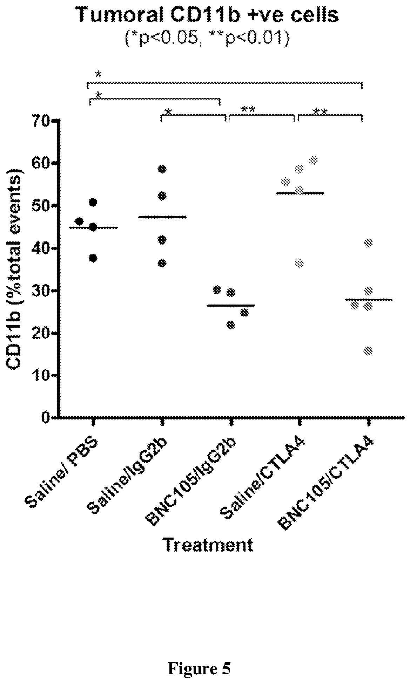

**Figure 5****.** Reduction in the number of tumor infiltrating macrophages (CD11b+) after treatment with BNC105 (monotherapy and combination).

### DETAILED DESCRIPTION

### General Techniques and Definitions

Unless specifically defined otherwise, all technical and scientific terms used herein shall be taken to have the same meaning as commonly understood by one of ordinary skill in the art (e.g., in chemistry, biochemistry, and immunology).

Unless otherwise indicated, the chemistry, biochemistry, and immunological techniques utilized in the present invention are standard procedures, well known to those skilled in the art. Such techniques are described and explained throughout the literature in sources such as, J, Perbal, A Practical Guide to Molecular Cloning, John Wiley and Sons (1984), J. Sambrook and Russell., Molecular Cloning: A Laboratory Manual, 3rd edn, Cold Spring Harbour Laboratory Press (2001), R. Scopes, Protein Purification - Principals and Practice, 3rd edn, Springer (1994), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

The term "and/or", e.g., "X and/or Y" shall be understood to mean either "X and Y" or "X or Y" and shall be taken to provide explicit support for both meanings or for either meaning.

As used herein, the term "about", unless stated to the contrary, refers to +/- 10% of the designated value.

As used herein, the terms "treating", "treat" or "treatment" include administering a vascular disrupting agent and an immunotherapeutic agent to a patient in an amount sufficient to prevent or delay disease progression and/or to increase the duration of progression free survival as compared to a patient who has not been administered the vascular disrupting agent and the immunotherapeutic agent.

As used herein, the terms "response", "responding", "response to treatment" or "responding to treatment" refer to a patient having a reduction in one or more symptoms or signs of disease and/or a delay or prevention of disease progression, and/or a longer period of disease free progression during and/or following treatment with a combination of a vascular disrupting agent and an immunotherapeutic agent when compared to a patient that has not been treated with the combination of the vascular disrupting agent and the immunotherapeutic agent.

"Administering" as used herein is to be construed broadly and includes administering a composition or therapeutic agent as described herein to a subject or patient as well as providing the composition or therapeutic agent to a cell, such as, for example, by the provision of a prodrug to a patient.

### Combination Treatment

In order to increase the efficacy of the treatment of cancer with vascular disrupting agents, studies are being conducted to define patient subpopulations that would most likely benefit from treatment with a vascular disrupting agent. In the current state of the art, however, there is no expectation that the efficacy of treating cancer with a vascular disrupting agent would be enhanced by combining it with an immunotherapeutic agent, particularly when the immunotherapeutic agent is an immune checkpoint inhibitor. The present inventors have now demonstrated the efficacy of a combination treatment comprising a vascular disrupting agent and an immunotherapeutic agent in the treatment of cancer. In particular, the inventors have determined that immune activation that results from changes to the tumor and its micro-environment following administration of a vascular disrupting agent provides a key opportunity to leverage a response from immunotherapeutic agents in tumors that would otherwise be tolerated by the immune system. Hence, this allows for the therapeutic value of immunotherapeutic agents to push deeper into patient populations generating a larger number of treatment 'responders'.

### Vascular Disrupting Agents

Endothelial cells are highly dependent on the tubulin cytoskeleton for their motility, invasion, attachment, alignment and proliferation. Vascular disrupting agents (VDAs) target endothelial cells and pericytes of the already established tumor vasculature. Most VDAs induce changes in endothelial cell shape by disruption of the cytoskeleton and cell-to-cell junctions. This results in increased permeability to proteins and an increased interstitial fluid pressure, which might be sufficient to reduce vessel diameter. Plasma leakage also leads to increased blood viscosity resulting in decreased blood flow and rouleaux formation.

Another factor contributing to the vascular shutdown is the activation of platelets through contact with basement membrane components, which are exposed. All together this cascade of events results in vascular shutdown more selectively in tumor endothelium than normal endothelium. As stated previously, it is suggested that the inhibition of blood flow and the subsequent compromised supply of oxygen and nutrients will induce necrosis of many tumor cells downstream.

Vascular disrupting agents have been divided into two types, small molecule VDAs and ligand directed VDAs. Small molecule VDAs are in a more advanced stage of clinical development. Small molecule VDAs include tubulin-binding agents and flavonoids. Tubulin-binding agents are proposed to act at the colchicine-binding site of the β-subunit of endothelial cell tubulin, resulting in depolymerization of microtubules and disorganization of actin and tubulin (e.g. CA4 (combretastatin)).

Disruption of the endothelial cytoskeleton results in cell morphology changes leading to reduction or cessation of blood flow. Tumor-related endothelial cells are much more sensitive to the activity of tubulin-binding agents than normal endothelial cells. ASA404 is a small-molecule flavonoid VDA with activity involving inhibition of pathways that up regulate the nuclear transcription factor NfκB and production of TNF-*α* and other cytokines.

Thus, in one embodiment, the vascular disrupting agent is a Tubulin Polymerization Inhibitor (TPI). As used herein the term "tubulin polymerisation inhibitor" refers to any and all compounds or molecules which directly interact with tubulin and inhibit tubulin polymerisation and/or depolymerise tubulin and as a consequence interferes with the physiological function of microtubules. Tubulin polymerisation inhibitors (TPIs) are also referred to as microtubule "destabilizing" agents. Such compounds should be contrasted with tubulin interacting compounds like taxanes and epothilones which stabilise tubulin polymers and inhibit tubulin depolymerisation (i.e., microtubule stabilising agents).

Microtubules are filamentous polymers that are key components of the cell cytoskeleton. They are dynamic structures fluctuating between states of polymerisation and depolymerisation. This property enables microtubules to modulate cell shape, adhesion, migration and proliferation. TPIs interfere with microtubule integrity, leading to cytoskeletal changes of the endothelial cells that line the blood vessels of the tumour. As a result, these usually flat cells become more rounded, and lose their cell to cell contact. These events lead to narrowing of tumour blood vessels and ultimately occlusion of blood flow through the vessels. TPIs directly disrupt microtubule polymerisation processes and consequently have the ability to effect cell shape changes and inhibit cell proliferation. These properties are central to the use of TPIs as therapeutics for the treatment of cancer.

TPIs may also be classified based on their specific tubulin binding site. Binding of vinca alkaloids to tubulin defines a site that mediates the tubulin destabilization activity seen with these compounds. The "vinca" site has been shown to directly bind a number of compounds that effect destabilization of tubulin. Examples of TPI's that bind to the vinca site include vinflunine, vinblastine, vincristine, vinorelbine, dolastatin, tasidotin and E7974.

Colchicine binding to tubulin defines an independent binding site that like in the case of the "vinca" site causes destabilization of tubulin. Although TPI's binding to the "vinca" sites have been successful as anti-cancer chemotherapeutics, "colchicine" site binders have been in comparison neglected, possibly due to the lack of therapeutic margins offered by colchicine. However, more recently a number of "colchicine" site binding agents have been described that have the ability to cause disruption of blood vessels within solid tumors. Many of the "colchicine" site binding agents are based on natural products such as combretastatins (CA4P, OXi-4503, AVE-8062), colchicines (ZD6126) and phenylahistin (NPI-2358) while others are small molecules which bind to the colchicine site (ABT-751, MPC-6827, AEZS-112, CYT-997, MN-029, EPC2407, ZIO-301, 2ME2, ZD6126 and NPI-2358).

TPI compounds are important in the treatment of cancers primarily as a result of their capacity to selectively shut down blood flow through a tumour. Targeting tubulin polymerisation inhibition has been a very well validated anti-cancer approach through the development and now extensive clinical use of chemotherapeutic TPIs.

Examples of TPIs described herein but not falling within the scope of the claimed invention include ABT-751 (E7010, Abbott), MPC-6827 (AzixaTM, Myriad Pharmaceuticals), AEZS-112 (ZEN-012, Eterna Zentaris), CYT997 (Cytopia), MN-029 (Denibulin, MediciNova/Angiogene), EPC2407 (EpiCept), ZIO-301 (Indibulin, Ziopharm Oncology), Vinflunine (Javlor, Pierre Fabre Medicament) as well as other vinca alkaloids (e.g., vinblastin, vincristine, and vinorelbine), combretastatins (CA4 (Zybrestat^{™}, OXiGENE), Oxi4503 (OXiGENE), and AVE8062 (AC7700, Sanofi Aventis)), Eribulin Mesylate (E7389, Eisai), Dolastatin 10 (NCI), Tasidotin (synthadotin, Genzyme), 2-methoxyestradiol (2ME2 or Panzem^{®}, EntreMed), E7974 (Eisai), and NPI-2358 (Nereus Pharmaceuticals). Examples of TPI structures not part of the claimed invention are provided in Table 1.

**Table 1. Examples of TPI structures not within the scope of the claimed invention**

| | |
|---|---|
| ABT-751 (E7010, Abbott) | Vinflunine |
| 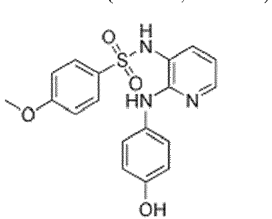 | 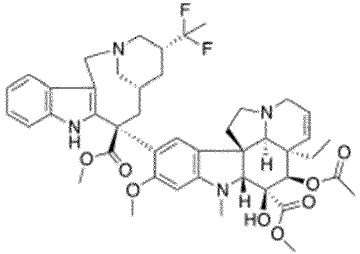 |
| MPC-6827 (Azixa^{™}, Myriad Pharmaceuticals) | Vinblastin |
| 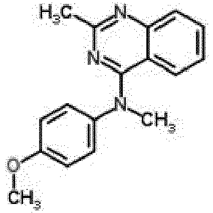 | 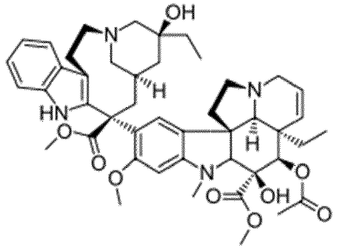 |
| AEZS-112 (ZEN-012, Eterna Zentaris) | Vincristine |
| 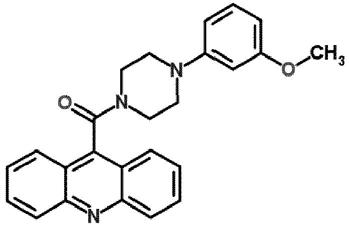 | 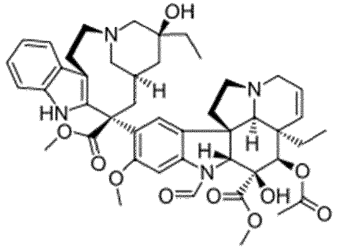 |
| CYT997 (Gilead) | Vinorelbine |
| 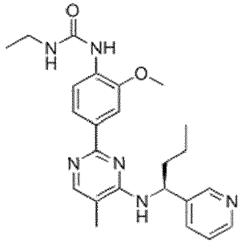 | 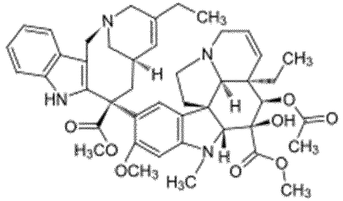 |
| MN-029 (Denibulin, MediciNoval/Angiogene) | Dolastatin 10 (NCI) |
| | 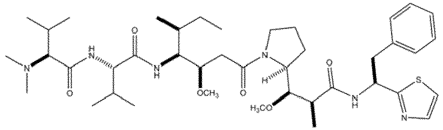 |
| 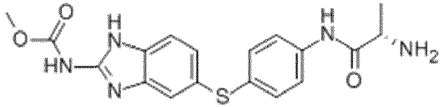 | |
| EPC2407 (EpiCept) | Tasidotin (synthadotin, Genzyme) H-Cl |
| 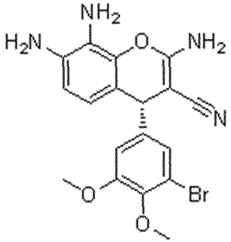 | |
| | 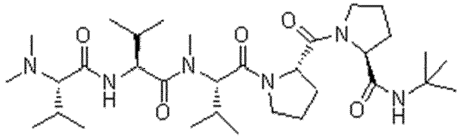 |
| ZIO-301 (Indibulin, Ziopharm Oncology) | E7974 (Eisai) |
| 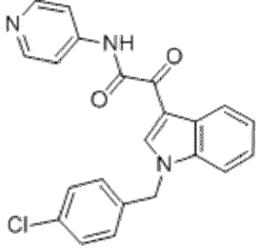 | 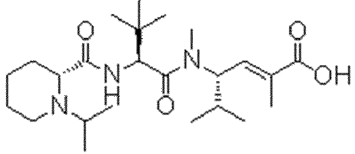 |
| CA4 (Zybrestat^{™}, OXiGENE) | Oxi4503 (OXiGENE) |
| 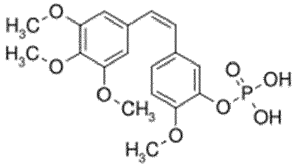 | 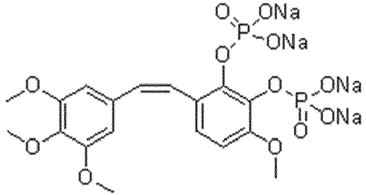 |
| AVE8062 (AC7700, Sanofi Aventis) | Eribulin Mesylate (E7389, Eisai) |
| 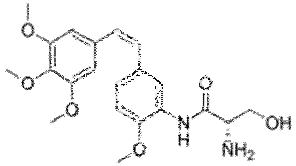 | 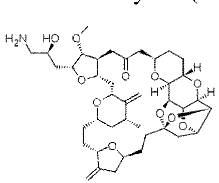 |
| 2-methoxyestradiol (2ME2 or Panzem^{®}, EntreMed) | |
| 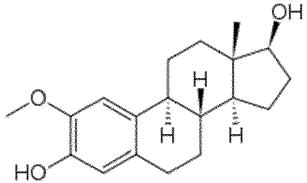 | |

In an embodiment described herein but not falling within the scope of the claimed invention, the TPI is selected from a compound of formula (I) or salts, solvates or prodrugs thereof 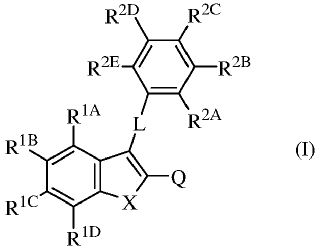 wherein;
X represents O, S, SO, SO₂, Se, SeO, SeO₂ or NR where R is selected from H, O, optionally substituted acyl, optionally substituted alkenyl, optionally substituted alkyl, optionally substituted aryl, optionally substituted cycloalkenyl, optionally substituted cycloalkyl, optionally substituted heteroaryl, optionally substituted heterocyclyl, and optionally substituted sulfonyl;
R^{1A} and R^{1B} each independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacyloxy, optionally substituted oxyacylimino, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or R^{1A} and R^{1B} together form an optionally substituted aryl, optionally substituted heterocyclyl, optionally substituted heteroaryl, optionally substituted cycloalkyl, or optionally substituted cycloalkenyl;
R^{1C} represents C₁₋₃ alkoxy, C₁₋₃ alkylthio, C₁₋₃ alkylamino, or C₁₋₃ dialkylamino;
R^{1D} represents hydroxy or amino;
L represents C=O, O, S, SO, SO₂, Se, SeO, SeOz, C=NZ', or NR' where Z' is H, optionally substituted alkyl, optionally substituted aryl or optionally substituted amino; and where R' is selected from H, O, optionally substituted acyl, optionally substituted alkenyl, optionally substituted alkyl, optionally substituted aryl, optionally substituted cycloalkenyl, optionally substituted cycloalkyl, optionally substituted heteroaryl, optionally substituted heterocyclyl, or optionally substituted sulfonyl;
R^{2A}-R^{2E} each independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacylimino, optionally substituted oxyacyloxy, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or optionally substituted thioacyloxy; or any of R^{2A} and R^{2B}, R^{2B} and R^{2C}, R^{2C} and R^{2D}, and R^{2D} and R^{2E}, together form an optionally substituted aryl, optionally substituted heterocyclyl, optionally substituted heteroaryl, optionally substituted cycloalkyl, or optionally substituted cycloalkenyl; and
Q represents H, CN, halogen, trialkylsilyl, optionally substituted alkyl, optionally substituted alkenyl, optionally substituted alkynyl, optionally substituted acyl, optionally substituted oxyacyl, optionally substituted acylamino, optionally substituted aminoacylamino, OR", SR" or NR"R", where each R" independently represents, H, optionally substituted alkyl, optionally substituted alkenyl, optionally substituted alkynyl, optionally substituted aryl, optionally substituted heteroaryl, optionally substituted heterocyclyl, optionally substituted acyl and optionally substituted oxyacyl, or NR‴NR‴, where each R‴ independently represents H, optionally substituted alkyl, optionally substituted alkenyl, optionally substituted alkynyl, optionally substituted aryl and optionally substituted heteroaryl.

In some embodiments X is selected from
O,
S,
SO,
SO₂,
Se,
SeO,
SeO₂ or
NR where R is selected from
H,
O,
optionally substituted acyl selected from H-C(O)-, C₁-C₁₀ alkyl-C(O)- (preferably C₁-C₆ alkyl, more preferably C₁-C₃ alkyl), C₄-C₈ cycloalkyl-C(O)-, C₆-C₁₄ aryl-C(O)-, heteroaryl-C(O)- having from 2 to 10 carbon atoms and 1 to 4 heteroatoms selected from oxygen, nitrogen, selenium, and sulfur (including oxides of sulfur, selenium and nitrogen) within the ring or heterocyclyl-C(O)- having from 1 to 8 carbon atoms and from 1 to 4 heteroatoms selected from nitrogen, sulfur, oxygen, selenium or phosphorous within the ring. Examples of suitable acyl groups include formyl acetyl, propionyl, benzoyl (optionally substituted with methyl, methoxy, halogen, nitro, trifluoromethyl or cyano);
optionally substituted monovalent C₂-C₁₀ alkenyl group which may be straight chained or branched (preferably C₂-C₆ alkenyl) having at least 1 or from 1-2 carbon to carbon double bonds. Examples of suitable optionally substituted alkenyl groups include, ethenyl, *n-*propenyl, iso-propenyl, but-2-enyl, 1-propenyl, vinyl, nitrovinyl, cyano vinyl, or trifluorovinyl and styryl (optionally substituted with methyl, methoxy, halogen, nitro, trifluoromethane or cyano);
optionally substituted C₁-C₁₀ alkyl (preferably C₁-C₆ alkyl, more preferably C₁-C₃ alkyl). Examples of suitable alkyl groups include methyl, ethyl, n-propyl, iso-propyl, n-butyl, iso-butyl, n-hexyl, 1-hydroxyethyl, 1-thioethyl, methoxyiminomethyl, ethoxyiminomethyl, 1-(hydroxyimino)ethyl, 1-(hydroxyimino)propyl, 1-hydrazinoethyl, 1-hydrazinopropyl, hydroxyiminomethyl, 2-oxopropyl, 2-oxobutyl, 3-oxobutyl, 3-oxopentyl, nitromethyl, 1-nitromethyl, and 2-nitroethyl;
optionally substituted C₆-C₁₄ aryl;
optionally substituted C₄-C₈ cycloalkenyl;
optionally substituted C₃-C₈ cycloalkyl;
optionally substituted heteroaryl having from 2 to 10 carbon atoms and 1 to 4 heteroatoms selected from oxygen, nitrogen, selenium, and sulfur (including oxides of sulfur, selenium and nitrogen) within the ring;
optionally substituted heterocyclyl having from 1 to 8 carbon atoms and from 1 to 4 heteroatoms selected from nitrogen, sulfur, oxygen, selenium or phosphorous within the ring; and
optionally substituted sulfonyl selected from H-S(O)₂-, C₁-C₁₀ alkyl-S(O)₂-(preferably C₁-C₆ alkyl, more preferably C₁-C₃ alkyl), C₃-C₈ cycloalkyl-S(O)₂-, C₆-C₁₄ aryl-S(O)₂-, heteroaryl-S(O)₂- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-S(O)₂- where the heterocyclyl group has from 1 to 8 carbon atoms and from 1 to 4 heteroatoms selected from nitrogen, sulfur, oxygen, selenium or phosphorous within the ring. Examples of sulfonyl groups include methylsulfonyl, ethylsulfonyl, benzenesulfonyl (optionally substituted with methyl, methoxy, halogen, nitro, trifluoromethane or cyano), methoxycarbo, trifluoromethane;
In some embodiments described herein but not part of the claimed invention, R^{1A}-R^{1B} and R^{2A}-R^{2E} are independently selected from the following groups:
   hydrogen;
   C₁-C₁₀ alkyl, preferably C₁-C₆ alkyl, more preferably C₁-C₃ alkyl. Examples of suitable alkyl groups include methyl, ethyl, n-propyl, iso-propyl, n-butyl, iso-butyl and *n-*hexyl;
   substituted C₁-C₁₀ alkyl group, preferably C₁-C₆ alkyl, more preferably C₁-C₃ alkyl. Examples of substituted alkyl groups include 1-hydroxyethyl, 1-thioethyl, methoxyiminomethyl, ethoxyiminomethyl, 1-(hydroxyimino)ethyl, 1-(hydroxyimino)propyl, 1-hydrazinoethyl, 1-hydrazinopropyl, hydroxyiminomethyl, 2-oxopropyl, 2-oxobutyl, 3-oxobutyl, 3-oxopentyl, nitromethyl, 1-nitromethyl, and 2-nitroethyl;
   optionally subtituted acyl group selected from H-C(O)-, C₁-C₁₀ alkyl-C(O)-(preferably C₁-C₆ alkyl, more preferably C₁-C₃ alkyl), C₃-C₈ cycloalkyl-C(O)-, C₆-C₁₄ aryl-C(O)-, heteroaryl-C(O)- where the heteroaryl group has from 2 to 10 carbon atoms and 1 to 4 heteroatoms selected from oxygen, nitrogen, selenium, and sulfur (including oxides of sulfur, selenium and nitrogen) within the ring) and heterocyclyl-C(O)- where the heterocyclyl group has from 1 to 8 carbon atoms and from 1 to 4 heteroatoms selected from nitrogen, sulfur, oxygen, selenium or phosphorous within the ring). Examples of acyl groups include formyl acetyl, propionyl, benzoyl (optionally substituted with methyl, methoxy, halogen, nitro, trifluoromethyl or cyano);
   optionally substituted C₁-C₁₀ alkoxy group, preferably C₁-C₆ alkoxy, more preferably C₁-C₃ alkoxy. Examples of suitable alkoxy groups include methoxy, ethoxy, n-propoxy, *iso-*propoxy, n-butoxy, tert-butoxy, sec-butoxy, n-pentoxy, n-hexoxy and 1,2-dimethylbutoxy;
   optionally substituted oxyacyl group selected from HOC(O)-, C₁-C₁₀ alkyl-OC(O)-(preferably preferably C₁-C₆ alkyl, more preferably C₁-C₃ alkyl), C₃-C₈ cycloalkyl-OC(O)-, C₆-C₁₄ aryl-OC(O)-, heteroaryl-OC(O)- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-OC(O)- where the heterocyclyl group has from 1 to 8 carbon atoms and from 1 to 4 heteroatoms selected from nitrogen, sulfur, oxygen, selenium or phosphorous within the ring. Examples of oxyacyl groups include methoxycarbonyl, ethoxycarbonyl, propoxycarbonyl, butyloxycarbonyl, isobutyloxycarbonyl;
   optionally substituted acyloxy group selected from -OC(O)-(C₁-C₁₀ alkyl) (preferably C₁-C₆ alkyl, more preferably C₁-C₃ alkyl), -OC(O)-(C₆-C₁₄ aryl), -C(O)O-heteroaryl where the heteroaryl group has from 2 to 10 carbon atoms and 1 to 4 heteroatoms selected from oxygen, nitrogen, selenium, and sulfur (including oxides of sulfur, selenium and nitrogen) within the ring, and -C(O)O-heterocyclyl where the heterocyclyl group has from 1 to 8 carbon atoms and from 1 to 4 heteroatoms selected from nitrogen, sulfur, oxygen, selenium or phosphorous within the ring. Examples of acyloxy groups include acetoxy and propioxy;
   optionally substituted (C₆-C₁₄ aryl)-( C₁-C₁₀ alkyl) group. Preferably the aryl group is C₆-C₁₀ aryl. Preferably the alkyl group is C₁-C₆ alkyl, more preferably C₁-C₃ alkyl. Examples of substituted arylalkyl groups include benzyl, phenethyl, 1-hydroxybenzyl, and 1-thiobenzyl;
   optionally substituted sulfinyl group selected from H-S(O)-, C₁-C₁₀ alkyl-S(O)-(preferably C₁-C₆ alkyl, more preferably C₁-C₃ alkyl), C₃-C₈ cycloalkyl-S(O)-, C₆-C₁₄ aryl-S(O)- (preferably, the aryl group has from 6 to 14 carbon atoms), heteroaryl-S(O)- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-S(O)- where the heterocyclyl group has from 1 to 8 carbon atoms and from 1 to 4 heteroatoms selected from nitrogen, sulfur, oxygen, selenium or phosphorous within the ring. Examples of sulfinyl groups include methylsulfinyl, ethylsulfinyl, benzene sulfinyl (optionally substituted with methyl, methoxy, halogen, nitro, trifluoromethane or cyano), methoxysulfinyl, ethoxysulfinyl;
   optionally substituted sulfonyl group selected from H-S(O)₂-, C₁-C₁₀ alkyl-S(O)₂-(preferably C₁-C₆ alkyl, more preferably C₁-C₃ alkyl), C₃-C₈ cycloalkyl-S(O)₂-, C₆-C₁₄ aryl-S(O)₂-, heteroaryl-S(O)₂- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-S(O)₂- where the heterocyclyl group has from 1 to 8 carbon atoms and from 1 to 4 heteroatoms selected from nitrogen, sulfur, oxygen, selenium or phosphorous within the ring. Examples of sulfonyl groups include methylsulfonyl, ethylsulfonyl, benzenesulfonyl (optionally substituted with methyl, methoxy, halogen, nitro, trifluoromethane or cyano), methoxycarbo, trifluoromethane;
   optionally substituted oxyacylamino group of the formula -NR*C(O)OR* where each R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring and heterocyclyl having from 1 to 8 carbon atoms and from 1 to 4 heteroatoms selected from nitrogen, sulfur, oxygen, selenium or phosphorous within the ring. Examples of oxyacylamino groups include methoxycarbonylamido, and ethoxycarbonyl amido;
   optionally substituted oxythioacyl group selected from HO-C(S)-, C₁-C₁₀ alkylO-C(S)- (preferably C₁-C₆ alkyl, more preferably C₁-C₃ alkyl), C₃-C₈ cycloalkylO-C(S)-, C₆-C₁₄ arylO-C(S)-, heteroarylO-C(S)- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclylO-C(S)- where the heterocyclyl group has from 1 to 8 carbon atoms and from 1 to 4 heteroatoms selected from nitrogen, sulfur, oxygen, selenium or phosphorous within the ring. Examples of oxythioacyl groups include methoxythiocarbonyl and ethoxythiocarbonyl;
   optionally substituted thioacyloxy group selected from H-C(S)-O-, C₁-C₁₀ alkyl-C(S)-O- (preferably C₁-C₆ alkyl, more preferably C₁-C₃ alkyl), C₃-C₈ cycloalkyl-C(S)-O-, C₆-C₁₄ aryl-C(S)-O-, heteroaryl-C(S)-O- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-C(S)-O- where the heterocyclyl group has from 1 to 8 carbon atoms and from 1 to 4 heteroatoms selected from nitrogen, sulfur, oxygen, selenium or phosphorous within the ring. Examples of thioacyloxy groups include thionoacetoxy and thionopropionoxy;
   optionally substituted sulfinylamino group selected from H-S(O)-NR*-, C₁-C₁₀ alkyl-S(O)-NR*- (preferably the alkyl groups are C₁-C₆ alkyl, more preferably C₁-C₃ alkyl), C₃-C₈ cycloalkyl-S(O)-NR*-, C₆-C₁₄ aryl-S(O)-NR*-, heteroaryl-S(O)-NR*- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-S(O)-NR*- where the heterocyclyl group has from 1 to 8 carbon atoms and from 1 to 4 heteroatoms selected from nitrogen, sulfur, oxygen, selenium or phosphorous within the ring. R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring, and heterocyclyl having from 1 to 8 carbon atoms and from 1 to 4 heteroatoms selected from nitrogen, sulfur, oxygen, selenium or phosphorous within the ring. Examples of sulfinylamino groups include methylsulfinylamino, ethylsulfinylamino, and benzenesulfinylamino (optionally substituted with methyl, methoxy, halogen, nitro, trifluoromethane or cyano);
   amino group;
   substituted amino groups of the formula -NR*R* where each R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring and heterocyclyl having from 1 to 8 carbon atoms and from 1 to 4 heteroatoms selected from nitrogen, sulfur, oxygen, selenium or phosphorous within the ring. Examples of substituted amino groups include residues of L-valine, D-valine, L-alanine, D-alanine, aspartic acid, and alanylserine, N-methylamino, and N,N'-dimethylamino;
   optionally substituted sulfonylamino group selected from H-S(O)₂-NR*-, C₁-C₁₀ alkyl-S(O)₂-NR*- (preferably C₁-C₆ alkyl, more preferably C₁-C₃ alkyl), C₃-C₈ cycloalkyl-S(O)₂-NR*-, C₆-C₁₄ aryl-S(O)₂-NR*-, heteroaryl-S(O)₂-NR*- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-S(O)2-NR*- where the heterocyclyl group has from 1 to 8 carbon atoms and from 1 to 4 heteroatoms selected from nitrogen, sulfur, oxygen, selenium or phosphorous within the ring. R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring and heterocyclyl having from 1 to 8 carbon atoms and from 1 to 4 heteroatoms selected from nitrogen, sulfur, oxygen, selenium or phosphorous within the ring. Examples of sulfonylamino groups include methylsulfonylamino, ethylsulfonylamino and benzene sulfonylamino (optionally substituted with methyl, methoxy, halogen, nitro, trifluoromethane or cyano);
   optionally substituted oxysulfinylamino group selected from HO-S(O)-NR*-, C₁-C₁₀ alkylO-S(O)-NR*- (preferably C₁-C₆ alkyl, more preferably C₁-C₃ alkyl), C₃-C₈ cycloalkylO-S(O)-NR*-, C₆-C₁₄ arylO-S(O)-NR*-, heteroarylO-S(O)-NR*- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclylO-S(O)-NR*- where the heterocyclyl group has from 1 to 8 carbon atoms and from 1 to 4 heteroatoms selected from nitrogen, sulfur, oxygen, selenium or phosphorous within the ring. R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring and heterocyclyl having from 1 to 8 carbon atoms and from 1 to 4 heteroatoms selected from nitrogen, sulfur, oxygen, selenium or phosphorous within the ring. Examples of suitable oxysulfinylamino groups include methoxysulfinylamino and ethoxysulfinylamino;
   optionally substituted oxysulfonylamino group selected from HO-S(O)₂-NR*-, C₁-C₁₀ alkylO-S(O)₂-NR*- (preferably C₁-C₆ alkyl, more preferably C₁-C₃ alkyl), C₃-C₈ cycloalkylO-S(O)₂-NR*-, C₆-C₁₄ arylO-S(O)₂-NR*-, heteroarylO-S(O)₂-NR*- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclylO-S(O)₂-NR*- where the heterocyclyl group has from 1 to 8 carbon atoms and from 1 to 4 heteroatoms selected from nitrogen, sulfur, oxygen, selenium or phosphorous within the ring. R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring and heterocyclyl having from 1 to 8 carbon atoms and from 1 to 4 heteroatoms selected from nitrogen, sulfur, oxygen, selenium or phosphorous within the ring. Examples of oxysulfonylamino groups include methoxysulfonylamino and ethoxysulfonylamino;
   optionally substituted C₂-C₁₀ alkenyl group which may be straight chained or branched and have at least 1 or from 1-2 carbon to carbon double bonds. Preferably, optionally substituted C₂-C₆ alkenyl. Examples of suitable optionally substituted alkenyl groups include ethenyl, n-propenyl, iso-propenyl, but-2-enyl, 1-propenyl, vinyl, nitrovinyl, cyano vinyl, or trifluorovinyl and styryl (optionally substituted with methyl, methoxy, halogen, nitro, trifluoromethane or cyano);
   optionally substituted C₂-C₁₀ alkynyl group having at least 1 or from 1-2 carbon to carbon triple bonds. Preferably C₂-C₆ alkynyl. Examples of suitable alkynyl groups include 1-propynyl, ethynyl, propargyl, pent-2-ynyl and trimethylsilylethynyl.

In some embodiments described herein but not part of the claimed invention, R^{1C} is selected from the following groups:
C₁₋₃ alkoxy. Examples of suitable alkoxy groups include methoxy, ethoxy, n-propoxy, and iso-propoxy;
C₁₋₃ alkylthio. Examples of suitable alkylthio groups include methyl-S-, ethyl-S-, 1-thio-propyl, 2-thio-propyl and iso-propyl-S-;
C₁₋₃ alkylamino. Examples of suitable alkylamino groups include methylamino, ethylamino, 1-amino-propyl, 2-amino-propyl, and iso-propyl-amino; and
C₁₋₃ dialkylamino. Examples of suitable alkylamino groups include dimethylamino, diethylamino, dipropylamino, ethylmethylamino, propylmethylamino, and propylmethylamino, where the alkyl groups may be straight chained or branched;

In some embodiments described hereinbut not part of the claimed invention, R^{1D} is selected from a hydroxy group and an amino group.

In some embodiments described herein but not part of the claimed invention, L is selected from the following groups:
C=O,
O,
S,
SO,
SO₂,
Se,
SeO,
SeO₂,
C=NZ' where Z' is H, optionally substituted C₁-C₁₀ alkyl (preferably C₁-C₆, more preferably C₁-C₃), optionally substituted C₆-C₁₄ aryl or optionally substituted amino, or
NR' where R' is selected from
H,
O,
optionally substituted acyl group selected from H-C(O)-, C₁-C₁₀ alkyl-C(O)-(preferably C₁-C₆ alkyl, more preferably C₁-C₃ alkyl), C₃-C₈ cycloalkyl-C(O)-, C₆-C₁₄ aryl-C(O)-, heteroaryl-C(O)- where the heteroaryl group has from 2 to 10 carbon atoms and 1 to 4 heteroatoms selected from oxygen, nitrogen, selenium, and sulfur (including oxides of sulfur, selenium and nitrogen) within the ring and heterocyclyl-C(O)- where the heterocyclyl group has from 1 to 8 carbon atoms and from 1 to 4 heteroatoms selected from nitrogen, sulfur, oxygen, selenium or phosphorous within the ring). Examples of acyl groups include formyl acetyl, propionyl, benzoyl (optionally substituted with methyl, methoxy, halogen, nitro, trifluoromethyl or cyano);
optionally substituted C₂-C₁₀ alkenyl group which may be straight chained or branched and have at least 1 or from 1-2 carbon to carbon double bonds. Preferably optionally substituted C₂-C₆ alkenyl. Examples of suitable optionally substituted alkenyl groups include ethenyl, n-propenyl, iso-propenyl, but-2-enyl, 1-propenyl, vinyl, nitrovinyl, cyano vinyl, or trifluorovinyl and styryl (optionally substituted with methyl, methoxy, halogen, nitro, trifluoromethane or cyano);
optionally substituted C₁-C₁₀ alkyl, preferably C₁-C₆ alkyl, more preferably C₁-C₃ alkyl. Examples of suitable alkyl groups include methyl, ethyl, 1-hydroxyethyl, 1-thioethyl, methoxyiminomethyl, ethoxyiminomethyl, 1-(hydroxyimino)ethyl, 1-(hydroxyimino)propyl, 1-hydrazinoethyl, 1-hydrazinopropyl, hydroxyiminomethyl, 2-oxopropyl, 2-oxobutyl, 3-oxobutyl, 3-oxopentyl, nitromethyl, 1-nitromethyl, and 2-nitroethyl;
optionally substituted C₆-C₁₄ aryl;
optionally substituted C₄-C₈ cycloalkenyl;
optionally substituted C₃-C₈ cycloalkyl;
optionally substituted heteroaryl having from 2 to 10 carbon atoms and 1 to 4 heteroatoms selected from oxygen, nitrogen, selenium, and sulfur (including oxides of sulfur, selenium and nitrogen) within the ring
optionally substituted heterocyclyl having from 1 to 8 carbon atoms and from 1 to 4 heteroatoms selected from nitrogen, sulfur, oxygen, selenium or phosphorous within the ring; or
optionally substituted sulfonyl selected from H-S(O)₂-, C₁-C₁₀ alkyl-S(O)₂-(preferably C₁-C₆ alkyl, more preferably C₁-C₃ alkyl), C₃-C₈ cycloalkyl-S(O)₂-, C₆-C₁₄ aryl-S(O)₂-, heteroaryl-S(O)₂- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-S(O)₂- where the heterocyclyl group has from 1 to 8 carbon atoms and from 1 to 4 heteroatoms selected from nitrogen, sulfur, oxygen, selenium or phosphorous within the ring. Examples of sulfonyl groups include methylsulfonyl, ethylsulfonyl, benzenesulfonyl (optionally substituted with methyl, methoxy, halogen, nitro, trifluoromethane or cyano), methoxycarbo, trifluoromethane;
In some embodiments described herein but not part of the claimed invention, Q is selected from the following groups:
   H;
   CN;
   halogen, preferably Br or Cl;
   trialkylsilyl, in which each alkyl group is independently C₁-C₁₀ alkyl (preferably C₁-C₆ alkyl, more preferably C₁-C₃ alkyl);
   optionally substituted C₁-C₁₀ alkyl (preferably C₁-C₆ alkyl, more preferably C₁-C₃ alkyl). Examples of suitable alkyl groups include methyl, ethyl, propyl, butyl, aminoalkyl, oxyacylaminoalkyl and oxysulphonylaminoalkyl;
   optionally substituted C₂-C₁₀ alkenyl group which may be straight chained or branched and have at least 1 or from 1-2 carbon to carbon double bonds. Preferably optionally substituted C₂-C₆ alkenyl. Examples of suitable optionally substituted alkenyl groups include ethenyl, n-propenyl, iso-propenyl, but-2-enyl, 1-propenyl, vinyl, nitrovinyl, cyano vinyl, or trifluorovinyl and styryl (optionally substituted with methyl, methoxy, halogen, nitro, trifluoromethane or cyano);
   optionally substituted C₂-C₁₀ alkynyl group having at least 1 or from 1-2 carbon to carbon triple bonds. Preferably C₂-C₆ alkynyl. Examples of suitable alkynyl groups include 1-propynyl, ethynyl, propargyl, pent-2-ynyl, trimethylsilylethynyl and 2-alkylethynyl.
   optionally substituted oxyacyl selected from HOC(O)-, C₁-C₁₀ alkyl-OC(O)-(preferably preferably C₁-C₆ alkyl, more preferably C₁-C₃ alkyl), C₃-C₈ cycloalkyl-OC(O)-, C₆-C₁₄ aryl-OC(O)-, heteroaryl-OC(O)- where the heteroaryl group has from 2 to 10 carbon atoms and 1 to 4 heteroatoms selected from oxygen, nitrogen, selenium, and sulfur (including oxides of sulfur, selenium and nitrogen) within the ring, and heterocyclyl-OC(O)- where the heterocyclyl group has from 1 to 8 carbon atoms and from 1 to 4 heteroatoms selected from nitrogen, sulfur, oxygen, selenium or phosphorous within the ring. Examples of oxyacyl groups include methoxycarbonyl, ethoxycarbonyl, propoxycarbonyl, butyloxycarbonyl, isobutyloxycarbonyl;
   optionally substituted acyl group selected from H-C(O)-, C₁-C₁₀ alkyl-C(O)-(preferably C₁-C₆ alkyl, more preferably C₁-C₃ alkyl), C₃-C₈ cycloalkyl-C(O)-, C₆-C₁₄ aryl-C(O)-, heteroaryl-C(O)- where the heteroaryl group has from 2 to 10 carbon atoms and 1 to 4 heteroatoms selected from oxygen, nitrogen, selenium, and sulfur (including oxides of sulfur, selenium and nitrogen) within the ring and heterocyclyl-C(O)- where the heterocyclyl group has from 1 to 8 carbon atoms and from 1 to 4 heteroatoms selected from nitrogen, sulfur, oxygen, selenium or phosphorous within the ring). Examples of acyl groups include formyl acetyl, propionyl, benzoyl (optionally substituted with methyl, methoxy, halogen, nitro, trifluoromethyl or cyano);
   optionally substituted acylamino of the formula -NR*C(O)R* where each R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring and heterocyclyl having from 1 to 8 carbon atoms and from 1 to 4 heteroatoms selected from nitrogen, sulfur, oxygen, selenium or phosphorous within the ring;
   optionally substituted aminoacylamino, of the formula -NR*C(O)NR*R* where each R* is independently hydrogen, C₁-C₁₀ alkyl (preferably C₁-C₆ alkyl, more preferably C₁-C₃ alkyl), C₃-C₈ cycloalkyl, C₆-C₁₄ aryl, heteroaryl having from 2 to 10 carbon atoms and 1 to 4 heteroatoms selected from oxygen, nitrogen, selenium, and sulfur (including oxides of sulfur, selenium and nitrogen) within the ring and heterocyclyl having from 1 to 8 carbon atoms and from 1 to 4 heteroatoms selected from nitrogen, sulfur, oxygen, selenium or phosphorous within the ring;
   OR , where R" is selected from H or an optionally substituted C₁-C₁₀ alkyl (preferably C₁-C₆ alkyl, more preferably C₁-C₃ alkyl). Examples of suitable OR" groups include hydroxy, methoxy, ethoxy, n-propoxy, iso-propoxy, n-butoxy, tert-butoxy, sec-butoxy, n-pentoxy, *n-*hexoxy and 1,2-dimethylbutoxy;
   NR"R", preferably R" is selected from H, heteroaryl having from 2 to 10 carbon atoms and 1 to 4 heteroatoms selected from oxygen, nitrogen, selenium, and sulfur (including oxides of sulfur, selenium and nitrogen) within the ring, amino, aminoC₁-C₁₀ alkyl (preferably C₁-C₆ alkyl, more preferably C₁-C₃ alkyl), hydroxyl, hydroxyC₁-C₁₀ alkyl (preferably C₁-C₆ alkyl, more preferably C₁-C₃ alkyl), C₁-C₁₀ alkoxy (preferably C₁-C₆ alkoxy, more preferably C₁-C₃ alkoxy), C₁-C₁₀alkoxy C₁-C₁₀alkyl, oxyacyl, oxyacylalkyl, oxyacylamino, oxyacylaminoalkyl, guanidine, guanidinoalkyl or an optionally substituted C₁-C₁₀ alkyl group (preferably C₁-C₆ alkyl, more preferably C₁-C₃ alkyl). Examples of suitable NR"R" groups include NH₂, alkylamino, dialkylamino, heteroarylamino, aminoalkylamino, hydroxyalkylamino, alkoxyalkylamino, oxyacylalkylamino, oxyacylaminoalkylamino, guanidinoalkylamino;
   SR , preferably R" is selected from H, heteroaryl having from 2 to 10 carbon atoms and 1 to 4 heteroatoms selected from oxygen, nitrogen, selenium, and sulfur (including oxides of sulfur, selenium and nitrogen) within the ring, amino, aminoC₁-C₁₀ alkyl (preferably C₁-C₆ alkyl, more preferably C₁-C₃ alkyl), hydroxyl, hydroxyC₁-C₁₀ alkyl (preferably C₁-C₆ alkyl, more preferably C₁-C₃ alkyl), C₁-C₁₀ alkoxy (preferably C₁-C₆ alkoxy, more preferably C₁-C₃ alkoxy), C₁-C₁₀alkoxy C₁-C₁₀alkyl, oxyacyl, oxyacylalkyl, oxyacylamino, oxyacylaminoalkyl, guanidine, guanidinoalkyl or an optionally substituted C₁-C₁₀ alkyl group (preferably C₁-C₆ alkyl, more preferably C₁-C₃ alkyl). Examples of suitable S'R" groups include alkylthio, aminoalkylthio, heteroarylthio, aminoalkylthio, hydroxyalkylthio, alkoxyalkylthio, oxyacylalkylthio, oxyacylaminoalkylthio, guanidinoalkylthio; hydrazine.

In the definitions of the groups X, R^{1A}-R^{1B}, Q, L and R^{2A}-R^{2E}, the term "optionally substituted" refers to a group which may or may not be further substituted or fused (so as to form a condensed polycyclic group) with one or more groups selected from hydroxy, acyl, alkyl, alkoxy, alkenyl, alkenyloxy, alkynyl, alkynyloxy, amino, aminoacyl, thio, arylalkyl, arylalkoxy, aryl, aryloxy, acylamino, cyano, halogen, nitro, sulfo, phosphono, phosphorylamino, phosphinyl, heteroaryl, heteroaryloxy, heterocyclyl, heterocyclyloxy, oxyacyl, oxime, oxime ether, hydrazone, -NHC(NH)NH₂, oxyacylamino, oxysulfonylamino, aminoacyloxy, trihalomethyl, trialkylsilyl, pentafluoroethyl, trifluoromethoxy, difluoromethoxy, trifluoromethanethio, trifluoroethenyl, mono- and di-alkylamino, mono-and di-(substituted alkyl)amino, mono- and di-arylamino, mono- and di-heteroarylamino, mono- and di-heterocyclyl amino, and unsymmetric di-substituted amines having different substituents selected from alkyl, aryl, heteroaryl and heterocyclyl, and the like.

In one embodiment described hereinbut not part of the claimed invention, R^{2D}, R^{2C}, and R^{2B} are methoxy and L is a carbonyl group (C=O).

Accordingly, in this embodiment the TPIs are represented by formula (Ia) or salts, solvates, or prodrugs thereof 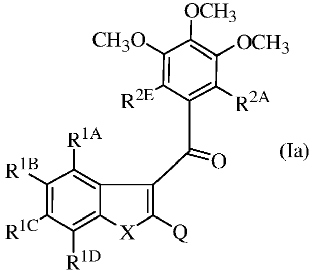 wherein;
X represents O, S, SO, SO₂, Se, SeO, SeO₂ or NR where R is selected from H, O, optionally substituted acyl, optionally substituted alkenyl, optionally substituted alkyl, optionally substituted aryl, optionally substituted cycloalkenyl, optionally substituted cycloalkyl, optionally substituted heteroaryl, optionally substituted heterocyclyl, and optionally substituted sulfonyl;
R^{1A} and R^{1B} each independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacyloxy, optionally substituted oxyacylimino, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or R^{1A} and R^{1B} together form an optionally substituted aryl, optionally substituted heterocyclyl, optionally substituted heteroaryl, optionally substituted cycloalkyl, or optionally substituted cycloalkenyl;
R^{1C} represents C₁₋₃ alkoxy, C₁₋₃ alkylthio, C₁₋₃ alkylamino, or C₁₋₃ dialkylamino;
R^{1D} represents hydroxy or amino;
R^{2A} and R^{2E} independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacyloxy, optionally substituted oxyacylimino, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or optionally substituted thioacyloxy; and
Q represents H, CN, halogen, trialkylsilyl, optionally substituted alkyl, optionally substituted alkenyl, optionally substituted alkynyl, optionally substituted acyl, optionally substituted oxyacyl, optionally substituted acylamino, optionally substituted aminoacylamino, OR", SR" or NR"R", where each R" independently represents, H, optionally substituted alkyl, optionally substituted alkenyl, optionally substituted alkynyl, optionally substituted aryl, optionally substituted heteroaryl, optionally substituted heterocyclyl, optionally substituted acyl and optionally substituted oxyacyl, or NR‴NR‴, where each R‴ independently represents H, optionally substituted alkyl, optionally substituted alkenyl, optionally substituted alkynyl, optionally substituted aryl and optionally substituted heteroaryl.

In another embodiment described herein but not part of the claimed invention, R^{1A}, R^{1B}, R^{2A} and R^{2E} represent H and R^{1C}, R^{2B}, R^{2C} and R^{2D} represents C₁₋₃ alkoxy.

Accordingly, in this embodiment the TPI is represented by formula (Ib) or salts, solvates or prodrugs thereof 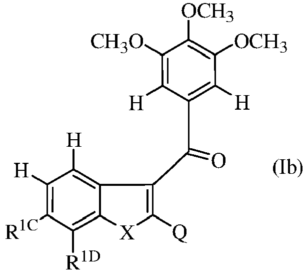 wherein;
X represents O, S, SO, SO₂, Se, SeO, SeO₂ or NR where R is selected from H, O, optionally substituted acyl, optionally substituted alkenyl, optionally substituted alkyl, optionally substituted aryl, optionally substituted cycloalkenyl, optionally substituted cycloalkyl, optionally substituted heteroaryl, optionally substituted heterocyclyl, and optionally substituted sulfonyl;
R^{1C} represents C₁₋₃ alkoxy;
R^{1D} represents hydroxy or amino;
   Q represents H, CN, halogen, trialkylsilyl, optionally substituted alkyl, optionally substituted alkenyl, optionally substituted alkynyl, optionally substituted acyl, optionally substituted oxyacyl, optionally substituted acylamino, optionally substituted aminoacylamino, OR", SR" or NR"R", where each R" independently represents, H, optionally substituted alkyl, optionally substituted alkenyl, optionally substituted alkynyl, optionally substituted aryl, optionally substituted heteroaryl, optionally substituted acyl and optionally substituted oxyacyl, or NR‴NR‴, where each R‴ independently represents H, optionally substituted alkyl, optionally substituted alkenyl, optionally substituted alkynyl, optionally substituted aryl and optionally substituted heteroaryl.

In a preferred embodiment described herein but not part of the claimed invention, R^{1C} represents methoxy.

For the compounds represented by formulae I, Ia and Ib, X is preferably selected from O, S and NR. More preferably X is O or NR and most preferably X is O.

Accordingly, in another embodiment described herein but not part of the claimed invention, the TPI is represented by formula II: 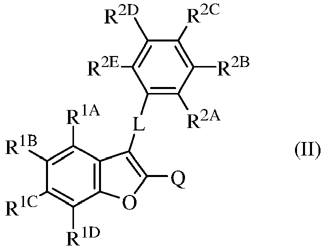 wherein;
R^{1A} and R^{1B} each independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacyloxy, optionally substituted oxyacylimino, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or R^{1A} and R^{1B} together form an optionally substituted aryl, optionally substituted heterocyclyl, optionally substituted heteroaryl, optionally substituted cycloalkyl, or optionally substituted cycloalkenyl;
R^{1C} represents C₁₋₃ alkoxy, C₁₋₃ alkylthio, C₁₋₃ alkylamino, or C₁₋₃ dialkylamino;
R^{1D} represents hydroxy or amino;
L represents C=O, O, S, SO, SO₂, Se, SeO, SeOz, C=NZ', or NR' where Z' is H, optionally substituted alkyl, optionally substituted aryl or optionally substituted amino; and where R' is selected from H, O, optionally substituted acyl, optionally substituted alkenyl, optionally substituted alkyl, optionally substituted aryl, optionally substituted cycloalkenyl, optionally substituted cycloalkyl, optionally substituted heteroaryl, optionally substituted heterocyclyl, or optionally substituted sulfonyl;
R^{2A}-R^{2E} each independently represents H, carboxy, cyano, dihalomethoxy, halogen, hydroxy, nitro, pentahaloethyl, phosphorylamino, phosphono, phosphinyl, sulfo, trihaloethenyl, trihalomethanethio, trihalomethoxy, trihalomethyl, optionally substituted acyl, optionally substituted acylamino, optionally substituted acylimino, optionally substituted acyliminoxy, optionally substituted acyloxy, optionally substituted arylalkyl, optionally substituted arylalkoxy, optionally substituted alkenyl, optionally substituted alkenyloxy, optionally substituted alkoxy, optionally substituted alkyl, optionally substituted alkynyl, optionally substituted alkynyloxy, optionally substituted amino, optionally substituted aminoacyl, optionally substituted aminoacyloxy, optionally substituted aminosulfonyl, optionally substituted aminothioacyl, optionally substituted aryl, optionally substituted aryloxy, optionally substituted cycloalkenyl, optionally substituted cycloalkyl, optionally substituted heteroaryl, optionally substituted heterocyclyl, optionally substituted oxyacyl, optionally substituted oxyacylamino, optionally substituted oxyacylimino, optionally substituted oxyacyloxy, optionally substituted oxysulfinylamino, optionally substituted oxysulfonylamino, optionally substituted oxythioacyl, optionally substituted oxythioacyloxy, optionally substituted sulfinyl, optionally substituted sulfinylamino, optionally substituted sulfonyl, optionally substituted sulphonylamino, optionally substituted thio, optionally substituted thioacyl, optionally substituted thioacylamino, or optionally substituted thioacyloxy; or any of R^{2A} and R^{2B}, R^{2B} and R^{2C}, R^{2C} and R^{2D}, and R^{2D} and R^{2E}, together form an optionally substituted aryl, optionally substituted heterocyclyl, optionally substituted heteroaryl, optionally substituted cycloalkyl, or optionally substituted cycloalkenyl; and
Q represents H, CN, halogen, trialkylsilyl, optionally substituted alkyl, optionally substituted alkenyl, optionally substituted alkynyl, optionally substituted acyl, optionally substituted oxyacyl, optionally substituted acylamino, optionally substituted aminoacylamino, OR", SR" or NR"R", where each R" independently represents, H, optionally substituted alkyl, optionally substituted alkenyl, optionally substituted alkynyl, optionally substituted heterocyclyl, optionally substituted aryl, optionally substituted heteroaryl, optionally substituted acyl and optionally substituted oxyacyl, or NR‴NR‴, where each R‴ independently represents H, optionally substituted alkyl, optionally substituted alkenyl, optionally substituted alkynyl, optionally substituted aryl and optionally substituted heteroaryl.

In this embodiment not being part of the claimed invention, it is preferred that L is a carbonyl group (C=O). Also, preferably at least one of R^{2D}, R^{2C} or R^{2B} represents a hydroxy or C₁₋₃ alkoxy group. More preferably when X=O, L is a carbonyl group an R^{2D}, R^{2C} and R^{2B} represent methoxy. Even more preferably when X=O, L is a carbonyl group, R^{2D}, R^{2C}, and R^{2B} represent methoxy and R^{1A}, R^{1B}, R^{2A}, R^{2E} are H.

Furthermore, for the compounds of formula (I), (Ia), (Ib) and (II) it is preferred that Q represents H, CN, optionally substituted C₂₋₄ alkynyl, optionally substituted C₂₋₆ alkenyl, optionally substituted C₁₋₄ alkyl, hydroxy, optionally substituted oxyacyl, NR"R", SR" (where each R" is independently H, optionally substituted C₁₋₄alkyl, optionally substituted heterocyclyl, optionally substituted heteroaryl), NR‴NR‴ (where each R‴ is independently H, C₁₋₃ alkyl), optionally substituted acylamino, or halogen.

In some embodiments described herein but not part of the claimed invention, Q is independently selected from the following groups:
H;
CN;
halogen, preferably Br or Cl;
alkyl group, preferably methyl, ethyl, propyl, butyl;
substituted alkyl group, preferably amino, oxyacylaminoalkyl and oxysulphonylaminoalkyl;
optionally substituted alkenyl, preferably ethenyl, 2-alkylethenyl, 2-oxyacylethenyl, 2-aminoacylethenyl;
optionally substituted alkynyl, preferably ethynyl, 2-alkylethynyl;
optionally substituted oxyacyl;
OR , preferably hydroxy, methoxy, ethoxy;
NR"R", preferably NH₂, alkylamino, dialkylamino, heteroarylamino, aminoalkylamino, hydroxyalkylamino, alkoxyalkylamino, oxyacylalkylamino, oxyacylaminoalkylamino, guanidinoalkylamino;
SR", preferably alkylthio, aminoalkylthio, heteroarylthio, aminoalkylthio, hydroxyalkylthio, alkoxyalkylthio, oxyacylalkylthio, oxyacylaminoalkylthio, guanidinoalkylthio; hydrazine.

In a further preferred embodiment described herein not part of the claimed invention, the TPI is a compound of formula (III) or a salt, solvate or prodrug thereof

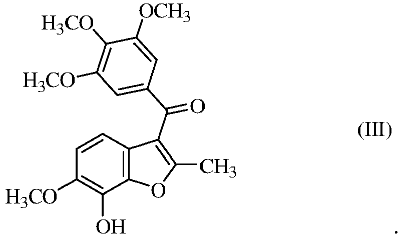

In an embodiment, the compound of formula (I), (Ia), (Ib), (II) or (III) is a prodrug selected from an ester, an acetate, a phosphate ester or an amide prodrug. In another embodiment, the compound of formula (I) (Ia), (Ib), (II) or (III) is a phosphate prodrug. In a particular embodiment, R^{1D} is hydroxy and the prodrug is a phosphate ester of the hydroxy group. Preferably, the phosphate ester is a disodium phosphate ester.

The compound of formula (III) (2-Methyl-7-hydroxy-3-(3,4,5-trimethoxybenzoyl)-6-methoxybenzofuran) can be prepared by the synthetic methodology described in PCT/AU2007/000101 (WO 07/087684).

The compounds of formula I, Ia, Ib, II or III have been observed to be potent tubulin polymerisation inhibitors (TPIs). An important aspect of the compounds of formulae I, Ia, Ib, II and III is the combination of the specific C-6 and C-7 substituents together with the C-2 Q-group (especially C-2 methyl) which appears to confer greater potency and selectivity when compared to other structurally related TPI compounds. In these compounds selectivity is not simply reliant on the predisposition of tumour vasculature towards collapse when challenged with the VDA but on a capacity of the VDA to distinguish between tumour endothelial cells and normal endothelial cells. Normal endothelial cells, found in healthy tissues, are in a "quiescent" state and tumour endothelial cells are in an "activated" state. Most VDAs do not distinguish between these two states, for example, Combretastatin A4 (CA4) is equally potent against quiescent and activated endothelial cells. However, the compounds of formulae I, Ia, Ib, II and particularly III show selectivity towards tumor endothelial cells (activated) over normal endothelial cells (quiescent).

In some embodiments described herein but not part of the claimed invention, the TPI for use in the present method is a compound of formula I, Ia, Ib or II or a salt, solvate or prodrug thereof wherein R^{1C} is C₁₋₃ alkoxy, R^{1D} is hydroxyl and Q is optionally substituted C₁₋₁₀ (or C₁₋₆ or C₁₋₃) alkyl.

The TPI compounds of formula I, Ia, Ib, II or III may be prepared by known methods including those disclosed in WO 02/060872 and WO 07/087684.

It will be appreciated that the TPIs and compounds of formula I, Ia, Ib, II, or III can be administered to a subject as a pharmaceutically acceptable salt thereof. Suitable pharmaceutically acceptable salts include, but are not limited to salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benezenesulphonic, salicyclic sulphanilic, aspartic, glutamic, edetic, stearic, palmitic, oleic, lauric, pantothenic, tannic, ascorbic and valeric acids.

Base salts include, but are not limited to, those formed with pharmaceutically acceptable cations, such as sodium, potassium, lithium, calcium, magnesium, ammonium and alkylammonium. In particular, the present invention includes within its scope cationic salts eg sodium or potassium salts, or alkyl esters (eg methyl, ethyl) of the phosphate group.

It will also be appreciated that any compound that is a prodrug of a TPI or a compound of formula I, Ia, Ib, II, and III not falling within the scope of the invention. The term "pro-drug" is used in its broadest sense and encompasses those derivatives that are converted *in vivo* to a compound (for instance, a compound of formulae I, Ia, Ib, II, and III). Such derivatives would readily occur to those skilled in the art, and include, for example, compounds where the free hydroxy group (for instance at C-7 position or R^{1D}) is converted into an ester, such as an acetate or phosphate ester, or where a free amino group (for instance at C-7 position or R^{1D}) is converted into an amide (e.g., α-amino acid amide). Procedures for esterifying, e.g. acylating, the compounds are well known in the art and may include treatment of the compound with an appropriate carboxylic acid, anhydride or chloride in the presence of a suitable catalyst or base. A particularly preferred prodrug is a disodium phosphate ester. The disodium phosphate ester (in particular a C-7 disodium phosphate ester of a compound of formula III) of the compound may be useful in increasing the solubility of the compounds. This, for instance, may allow for delivery of the compound in a benign vehicle like saline. The disodium phosphate ester may be prepared in accordance with the methodology described in Pettit et al. (1995). Other texts which generally describe prodrugs (and the preparation thereof) include: Design of Prodrugs, 1985, H. Bundgaard (Elsevier); The Practice of Medicinal Chemistry, 1996, Camille G. Wermuth et al., Chapter 31 (Academic Press); and A Textbook of Drug Design and Development, 1991, Bundgaard et al., Chapter 5, (Harwood Academic Publishers).

In some embodiments described herein but not within the scope of the claimed invention, the TPI is a compound of formula (IV) 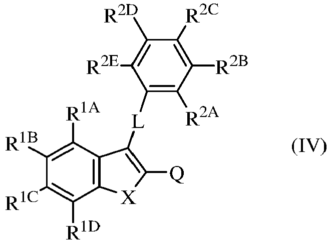 wherein, X, R^{1A}-R^{1C} and R^{2A}-R^{2E}, L and Q are as defined in formula I, Ia, Ib, II or III, and R^{1D} is OR³ or NHR³, and R³ is H or an ester. When R³ is an ester, the ester may consist of a carbonyl adjacent to an ether linkage (such as an acetate ester), or may be an inorganic ester (such as a phosphate, sulfate, nitrate or borate ester). In some embodiments, the ester is an acetate or a phosphate ester. A particularly preferred ester is a disodium phosphate ester.

The compounds of formulae I, Ia, Ib, II, and III (or a salt or prodrug thereof) may be in crystalline form either as the free compound or as a solvate (e.g. hydrate). Methods of solvation are generally known within the art.

### Chemical Definitions

"Alkyl" refers to monovalent alkyl groups which may be straight chained or branched and preferably have from 1 to 10 carbon atoms or more preferably 1 to 6 carbon atoms, and even more preferably 1 to 3 carbon atoms. Examples of such alkyl groups include methyl, ethyl, n-propyl, iso-propyl, n-butyl, iso-butyl, n-hexyl, and the like.

"Alkylene" refers to divalent alkyl groups preferably having from 1 to 10 carbon atoms and more preferably 1 to 6 carbon atoms, and even more preferably 1 to 3 carbon atoms. Examples of such alkylene groups include methylene (-CH₂-), ethylene (-CH₂CH₂-), and the propylene isomers (e.g., -CH₂CH₂CH₂- and -CH(CH₃)CH₂-), and the like.

"Aryl" refers to an unsaturated aromatic carbocyclic group having a single ring (eg., phenyl) or multiple condensed rings (eg., naphthyl or anthryl), preferably having from 6 to 14 carbon atoms. Examples of aryl groups include phenyl, naphthyl and the like.

"Arylene" refers to a divalent aryl group wherein the aryl group is as described above.

"Aryloxy" refers to the group aryl-O- wherein the aryl group is as described above.

"Arylalkyl" refers to -alkylene-aryl groups preferably having from 1 to 10 carbon atoms in the alkylene moiety and from 6 to 10 carbon atoms in the aryl moiety. Such arylalkyl groups are exemplified by benzyl, phenethyl and the like.

"Arylalkoxy" refers to the group arylalkyl-O- wherein the arylalkyl group are as described above. Such arylalkoxy groups are exemplified by benzyloxy and the like.

"Alkoxy" refers to the group alkyl-O- where the alkyl group is as described above. Examples include, methoxy, ethoxy, n-propoxy, iso-propoxy, n-butoxy, tert-butoxy, *sec-*butoxy, n-pentoxy, n-hexoxy, 1,2-dimethylbutoxy, and the like.

"Alkenyl" refers to a monovalent alkenyl group which may be straight chained or branched and preferably have from 2 to 10 carbon atoms and more preferably 2 to 6 carbon atoms and have at least 1 and preferably from 1-2, carbon to carbon, double bonds. Examples include ethenyl (-CH=CH₂), n-propenyl (-CH₂CH=CH₂), iso-propenyl (-C(CH₃)=CH₂), but-2-enyl (-CH₂CH=CHCH₃), and the like.

"Alkenyloxy" refers to the group alkenyl-O- wherein the alkenyl group is as described above.

"Alkenylene" refers to divalent alkenyl groups preferably having from 2 to 8 carbon atoms and more preferably 2 to 6 carbon atoms. Examples include ethenylene (-CH=CH-), and the propenylene isomers (e.g., -CH₂CH=CH- and -C(CH₃)=CH-), and the like.

"Alkynyl" refers to alkynyl groups preferably having from 2 to 10 carbon atoms and more preferably 2 to 6 carbon atoms and having at least 1, and preferably from 1-2, carbon to carbon, triple bonds. Examples of alkynyl groups include ethynyl (-C≡ CH), propargyl (-CH₂C≡ CH), pent-2-ynyl (-CH₂C≡CCH₂-CH₃), and the like.

"Alkynyloxy" refers to the group alkynyl-O- wherein the alkynyl groups is as described above.

"Alkynylene" refers to the divalent alkynyl groups preferably having from 2 to 8 carbon atoms and more preferably 2 to 6 carbon atoms. Examples include ethynylene (-C≡ C-), propynylene (-CH₂-C≡C-), and the like.

"Acyl" refers to groups H-C(O)-, alkyl-C(O)-, cycloalkyl-C(O)-, aryl-C(O)-, heteroaryl-C(O)- and heterocyclyl-C(O)-, where alkyl, cycloalkyl, aryl, heteroaryl and heterocyclyl are as described herein.

"Oxyacyl" refers to groups HOC(O)-, alkyl-OC(O)-, cycloalkyl-OC(O)-, aryl-OC(O)-, heteroaryl-OC(O)-, and heterocyclyl-OC(O)-, where alkyl, cycloalkyl, aryl, heteroaryl and heterocyclyl are as described herein.

"Amino" refers to the group -NR*R* where each R* is independently hydrogen, alkyl, cycloalkyl, aryl, heteroaryl, and heterocyclyl and where each of alkyl, cycloalkyl, aryl, heteroaryl and heterocyclyl is as described herein.

"Aminoacyl" refers to the group -C(O)NR*R* where each R* is independently hydrogen, alkyl, cycloalkyl, aryl, heteroaryl, and heterocyclyl and where each of alkyl, cycloalkyl, aryl, heteroaryl and heterocyclyl is as described herein.

"Aminoacylamino" refers to the group -NR*C(O)NR*R* where each R* is independently hydrogen, alkyl, cycloalkyl, aryl, heteroaryl, and heterocyclyl and where each of alkyl, cycloalkyl, aryl, heteroaryl and heterocyclyl is as described herein.

"Acylamino" refers to the group -NR*C(O)R* where each R* is independently hydrogen, alkyl, cycloalkyl, aryl, heteroaryl and heterocyclyl and where each of alkyl, cycloalkyl, aryl, heteroaryl, and heterocyclyl are as described herein.

"Acyloxy" refers to the groups -OC(O)-alkyl, -OC(O)-aryl, -C(O)O-heteroaryl, and
-C(O)O-heterocyclyl where alkyl, aryl, heteroaryl and heterocyclyl are as described herein.

"Aminoacyloxy" refers to the groups -OC(O)NR*-alkyl, -OC(O)NR*-aryl, -OC(O)NR*-heteroaryl, and -OC(O)NR*-heterocyclyl where R* is independently hydrogen, alkyl, cycloalkyl, aryl, heteroaryl, and heterocyclyl and where each of alkyl, cycloalkyl, aryl, heteroaryl and heterocyclyl is as described herein.

"Oxyacylamino" refers to the groups -NR*C(O)O-alkyl, -NR*C(O)O-aryl, -NR*C(O)O-heteroaryl, and NR*C(O)O-heterocyclyl where R* is independently hydrogen, alkyl, cycloalkyl, aryl, heteroaryl, and heterocyclyl and where each of alkyl, cycloalkyl, aryl, heteroaryl and heterocyclyl is as described herein.

"Oxyacyloxy" refers to the groups -OC(O)O-alkyl, -O-C(O)O-aryl, -OC(O)O-heteroaryl, and -OC(O)O-heterocyclyl where alkyl, cycloalkyl, aryl, heteroaryl, and heterocyclyl are as described herein.

"Acylimino" refers to the groups -C(NR*)-R* where each R* is independently hydrogen, alkyl, cycloalkyl, aryl, heteroaryl and heterocyclyl and where each of alkyl, cycloalkyl, aryl, heteroaryl, and heterocyclyl are as described herein.

"Acyliminoxy" refers to the groups -O-C(NR*)-R* where each R* is independently hydrogen, alkyl, cycloalkyl, aryl, heteroaryl and heterocyclyl and where each of alkyl, cycloalkyl, aryl, heteroaryl, and heterocyclyl are as described herein.

"Oxyacylimino" refers to the groups -C(NR*)-OR* where each R* is independently hydrogen, alkyl, cycloalkyl, aryl, heteroaryl and heterocyclyl and where each of alkyl, cycloalkyl, aryl, heteroaryl, and heterocyclyl are as described herein.

"Cycloalkyl" refers to cyclic alkyl groups having a single cyclic ring or multiple condensed rings, preferably incorporating 3 to 8 carbon atoms. Such cycloalkyl groups include, by way of example, single ring structures such as cyclopropyl, cyclobutyl, cyclopentyl, cyclohexyl, cyclooctyl, and the like, or multiple ring structures such as adamantanyl, and the like.

"Cycloalkenyl" refers to cyclic alkenyl groups having a single cyclic ring and at least one point of internal unsaturation, preferably incorporating 4 to 8 carbon atoms. Examples of suitable cycloalkenyl groups include, for instance, cyclobut-2-enyl, cyclopent-3-enyl, cyclohex-4-enyl, cyclooct-3-enyl and the like.

"Halo" or "halogen" refers to fluoro, chloro, bromo and iodo.

"Heteroaryl" refers to a monovalent aromatic heterocyclic group which fulfils the Hückel criteria for aromaticity (ie. contains 4n + 2 π electrons) and preferably has from 2 to 10 carbon atoms and 1 to 4 heteroatoms selected from oxygen, nitrogen, selenium, and sulfur within the ring (and includes oxides of sulfur, selenium and nitrogen). Such heteroaryl groups can have a single ring (eg., pyridyl, pyrrolyl or N-oxides thereof or furyl) or multiple condensed rings (eg., indolizinyl, benzoimidazolyl, coumarinyl, quinolinyl, isoquinolinyl or benzothienyl).

"Heterocyclyl" refers to a monovalent saturated or unsaturated group having a single ring or multiple condensed rings, preferably from 1 to 8 carbon atoms and from 1 to 4 heteroatoms selected from nitrogen, sulfur, oxygen, selenium or phosphorous within the ring. The most preferred heteroatom is nitrogen.

Examples of heterocyclyl and heteroaryl groups include, but are not limited to, oxazole, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, isothiazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiadiazoles, oxadiazole, oxatriazole, tetrazole, thiazolidine, thiophene, benzo[b]thiophene, morpholino, piperidinyl, pyrrolidine, tetrahydrofuranyl, triazole, and the like.

"Heteroarylene" refers to a divalent heteroaryl group wherein the heteroaryl group is as described above.

"Heterocyclylene" refers to a divalent heterocyclyl group wherein the heterocyclyl group is as described above.

"Thio" refers to groups H-S-, alkyl-S-, cycloalkyl-S-, aryl-S-, heteroaryl-S-, and heterocyclyl-S-, where alkyl, cycloalkyl, aryl, heteroaryl and heterocyclyl are as described herein.

"Thioacyl" refers to groups H-C(S)-, alkyl-C(S)-, cycloalkyl-C(S)-, aryl-C(S)-, heteroaryl-C(S)-, and heterocyclyl-C(S)-, where alkyl, cycloalkyl, aryl, heteroaryl and heterocyclyl are as described herein.

"Oxythioacyl" refers to groups HO-C(S)-, alkylO-C(S)-, cycloalkylO-C(S)-, arylO-C(S)-, heteroarylO-C(S)-, and heterocyclylO-C(S)-, where alkyl, cycloalkyl, aryl, heteroaryl and heterocyclyl are as described herein.

"Oxythioacyloxy" refers to groups HO-C(S)-O-, alkylO-C(S)-O-, cycloalkylO-C(S)-O-, arylO-C(S)-O-, heteroarylO-C(S)-O-, and heterocyclylO-C(S)-O-, where alkyl, cycloalkyl, aryl, heteroaryl and heterocyclyl are as described herein.

"Phosphorylamino" refers to the groups -NR*-P(O)(R**)(OR***) where R* represents H, alkyl, cycloalkyl, alkenyl, or aryl, R** represents OR*** or is hydroxy or amino and R*** is alkyl, cycloalkyl, aryl or arylalkyl, where alkyl, amino, alkenyl, aryl, cycloalkyl, and arylalkyl are as described herein.

"Thioacyloxy" refers to groups H-C(S)-O-, alkyl-C(S)-O-, cycloalkyl-C(S)-O-, aryl-C(S)-O-, heteroaryl-C(S)-O-, and heterocyclyl-C(S)-O-, where alkyl, cycloalkyl, aryl, heteroaryl, and heterocyclyl are as described herein.

"Sulfinyl" refers to groups H-S(O)-, alkyl-S(O)-, cycloalkyl-S(O)-, aryl-S(O)-, heteroaryl-S(O)-, and heterocyclyl-S(O)-, where alkyl, cycloalkyl, aryl, heteroaryl and heterocyclyl are as described herein.

"Sulfonyl" refers to groups H-S(O)z-, alkyl-S(O)₂-, cycloalkyl-S(O)₂-, aryl-S(O)₂-, heteroaryl-S(O)₂-, and heterocyclyl-S(O)₂-, where alkyl, cycloalkyl, aryl, heteroaryl and heterocyclyl are as described herein.

"Sulfinylamino" refers to groups H-S(O)-NR*-, alkyl-S(O)-NR*-, cycloalkyl-S(O)-NR*-, aryl-S(O)-NR*-, heteroaryl-S(O)-NR*-, and heterocyclyl-S(O)-NR*-, where R* is independently hydrogen, alkyl, cycloalkyl, aryl, heteroaryl, and heterocyclyl and where each of alkyl, cycloalkyl, aryl, heteroaryl and heterocyclyl is as described herein.

"Sulfonylamino" refers to groups H-S(O)₂-NR*-, alkyl-S(O)₂-NR*-, cycloalkyl-S(O)₂-NR*-, aryl-S(O)₂-NR*-, heteroaryl-S(O)₂-NR*-, and heterocyclyl-S(O)₂-NR*-, where R* is independently hydrogen, alkyl, cycloalkyl, aryl, heteroaryl, and heterocyclyl and where each of alkyl, cycloalkyl, aryl, heteroaryl and heterocyclyl is as described herein.

"Oxysulfinylamino" refers to groups HO-S(O)-NR*-, alkylO-S(O)-NR*-, cycloalkylO-S(O)-NR*-, arylO-S(O)-NR*-, heteroarylO-S(O)-NR*-, and heterocyclylO-S(O)-NR*-, where R* is independently hydrogen, alkyl, cycloalkyl, aryl, heteroaryl, and heterocyclyl and where each of alkyl, cycloalkyl, aryl, heteroaryl and heterocyclyl is as described herein.

"Oxysulfonylamino" refers to groups HO-S(O)₂-NR*-, alkylO-S(O)₂-NR*-, cycloalkylO-S(O)₂-NR*-, arylO-S(O)₂-NR*-, heteroarylO-S(O)₂-NR*-, and heterocyclylO-S(O)₂-NR*-, where R* is independently hydrogen, alkyl, cycloalkyl, aryl, heteroaryl, and heterocyclyl and where each of alkyl, cycloalkyl, aryl, heteroaryl and heterocyclyl is as described herein.

"Aminothioacyl" refers to groups R*R*N-C(S)-, where each R* is independently hydrogen, alkyl, cycloalkyl, aryl, heteroaryl, and heterocyclic and where each of alkyl, cycloalkyl, aryl, heteroaryl and heterocyclyl is as described herein.

"Thioacylamino" refers to groups H-C(S)-NR*-, alkyl-C(S)-NR*-, cycloalkyl-C(S)-NR*-, aryl-C(S)-NR*-, heteroaryl-C(S)-NR*-, and heterocyclyl-C(S)-NR*-, where R* is independently hydrogen, alkyl, cycloalkyl, aryl, heteroaryl, and heterocyclyl and where each of alkyl, cycloalkyl, aryl, heteroaryl and heterocyclyl is as described herein.

"Aminosulfinyl" refers to groups R*R*N-S(O)-, where each R* is independently hydrogen, alkyl, cycloalkyl, aryl, heteroaryl, and heterocyclic and where each of alkyl, cycloalkyl, aryl, heteroaryl and heterocyclyl is as described herein.

"Aminosulfonyl" refers to groups R*R*N-S(O)₂-, where each R* is independently hydrogen, alkyl, cycloalkyl, aryl, heteroaryl, and heterocyclic and where each of alkyl, cycloalkyl, aryl, heteroaryl and heterocyclyl is as described herein.

In this specification "optionally substituted" is taken to mean that a group may or may not be further substituted or fused (so as to form a condensed polycyclic group) with one or more groups selected from hydroxy, acyl, alkyl, alkoxy, alkenyl, alkenyloxy, alkynyl, alkynyloxy, amino, aminoacyl, thio, arylalkyl, arylalkoxy, aryl, aryloxy, acylamino, cyano, halogen, nitro, sulfo, phosphono, phosphorylamino, phosphinyl, heteroaryl, heteroaryloxy, heterocyclyl, heterocyclyloxy, oxyacyl, oxime, oxime ether, hydrazone, - NHC(NH)NH₂, oxyacylamino, oxysulfonylamino, aminoacyloxy, trihalomethyl, trialkylsilyl, pentafluoroethyl, trifluoromethoxy, difluoromethoxy, trifluoromethanethio, trifluoroethenyl, mono- and di-alkylamino, mono-and di-(substituted alkyl)amino, mono-and di-arylamino, mono- and di-heteroarylamino, mono- and di-heterocyclyl amino, and unsymmetric di-substituted amines having different substituents selected from alkyl, aryl, heteroaryl and heterocyclyl, and the like. An optionally substituted amino group may also include amino acid and peptide residues.

### Immunotherapeutic Agents

As used herein, the term "immunotherapeutic agent" refers to any therapeutic approach intended to mobilise, manipulate, up-regulate or disinhibit a patient's immune system to treat cancer. In one embodiment, Immunotherapy includes targeting of tumor cells via the recognition of immunogenic proteins or antigens expressed by said tumor cells, which may be accomplished by utilizing either passively transferred immune molecules such as antibodies, or cancer vaccine preparations designed to induce antibodies or T lymphocytes (T cells) recognizing a localized region of an antigen or epitope specific to the tumor cell.

In another embodiment, immunotherapy includes cellular therapies in which a patient's own immune cells are reprogrammed to attack the patient's cancer cells. By way of example, dendritic cell therapy provokes anti-tumor responses by causing dendritic cells to present tumor antigens. An FDA approved cellular therapy is Sipuleucel-T (Provenge, Dendreon, USA). One method of inducing dendritic cells to present tumor antigens is vaccination with short peptides. These peptides may be delivered with an adjuvant in order to induce a strong immune response, and a robust anti-tumor response by the immune system. Another strategy is to remove dendritic cells from the blood of a patient and activate them outside the body (*ex vivo*) in the presence of tumor antigens. The tumor antigens may be a single tumor-specific peptide/protein or a tumor cell lysate. These activated dendritic cells are put back into the body where they provoke an immune response to the cancer cells. Dendritic cell therapies include the use of antibodies that bind to the surface of dendritic cells. Antigens can be added to the antibody and can induce the dendritic cells to mature and provide immunity to the tumor. Dendritic cell receptors that have been used as targets by antibodies to produce immune responses include TLR3, TLR7, TLR8 and CD40.

In another embodiment, the immunotherapeutic agent is an antibody therapy. There are a number of antibody therapies approved for the treatment of cancer. Cell-surface receptors are common targets for antibody therapies and include, by way of non-limiting example, epidermal growth factor receptor and HER2. Once bound to a cancer antigen, antibodies can induce antibody-dependent cell-mediated cytotoxicity, activate the complement system, prevent a receptor interacting with its ligand and/or deliver a payload of chemotherapy or radiation, all of which can lead to cell death. In one embodiment, the antibody therapy is selected from bevacizumab, cetuximab, panitumumab, and trastuzumab.

In yet another embodiment according to the invention, the immunotherapeutic agent is an immune checkpoint inhibitor. "Immune checkpoint inhibitor," as used herein, refers to any compound or agent that inhibits the activity of an immune checkpoint protein. Immune checkpoint inhibitors can include, but are not limited to, immune checkpoint molecule binding proteins, antibodies (or fragments or variants thereof) that bind to immune checkpoint molecules, nucleic acids that down-regulate expression of the immune checkpoint molecules, or any other molecules that bind to immune checkpoint molecules (i.e. small organic molecules, peptidomimetics, aptamers, etc.) and which inhibit the function and/or activity of the immune checkpoint protein.

In one embodiment within the scope of the invention, the immune checkpoint inhibitor is selected from an inhibitor of: Programmed Death-Ligand 1 (PD-L1, also known as B7-H1, CD274), Programmed Death 1 (PD-1), CTLA-4, and described herein but not part of the invention PD-L2 (B7-DC, CD273), LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, GITR, HAVCR2, HVEM, IDO1, IDO2, ICOS (inducible T cell costimulator), KIR, LAIR1, LIGHT, MARCO (macrophage receptor with collageneous structure), PS (phosphatidylserine), OX-40, SLAM, TIGHT, VISTA, and/or VTCN1.

In some embodiments according to the invention, the immune checkpoint inhibitor is an inhibitor of PD-1. In one embodiment, the according to the invention immune checkpoint inhibitor is an anti-PD-1 antibody. In one particular embodiment, the immune checkpoint inhibitor is nivolumab. For example, the inhibitors of PD-1 biological activity (or its ligands) disclosed in U.S. Pat. Nos. 7,029,674; 6,808,710; or U.S. Patent Application Nos: 20050250106 and 20050159351 can be used in the methods provided herein. Exemplary antibodies against PD-1 include: Anti-mouse PD-1 antibody Clone J43 (Cat #BE0033-2) from BioXcell; Anti-mouse PD-1 antibody Clone RMP1-14 (Cat #BE0146) from BioXcell; mouse anti-PD-1 antibody Clone EH12; Merck's MK-3475 anti-mouse PD-1 antibody (Keytruda, pembrolizumab, lambrolizumab); and AnaptysBio's anti-PD-1 antibody, known as ANB011; antibody MDX-1 106 (ONO-4538); Bristol-Myers Squibb's human IgG4 monoclonal antibody nivolumab (Opdivo^{®}, BMS-936558, MDX1106); AstraZeneca's AMP-514, and AMP-224; and Pidilizumab (CT-011), CureTech Ltd.

In some embodiments according to the invention, the immune checkpoint inhibitor is an inhibitor of PD-L1. Exemplary immune checkpoint inhibitors include antibodies (e.g., an anti-PD-L1 antibody), RNAi molecules (e.g., anti-PD-L1 RNAi), antisense molecules (e.g., an anti-PD-L1 antisense RNA), dominant negative proteins (e.g., a dominant negative PD-L1 protein), and small molecule inhibitors. An exemplary anti-PD-L1 antibody includes clone EH12. Exemplary antibodies against PD-L1 include: Genentech's MPDL3280A (RG7446); Anti-mouse PD-L1 antibody Clone 10F.9G2 (Cat #BE0101) from BioXcell; anti-PD-L1 monoclonal antibody MDX-1105 (BMS-936559) and BMS-935559 from Bristol-Meyer's Squibb; MSB0010718C; mouse anti-PD-L1 Clone 29E.2A3; and AstraZeneca's MEDI4736.

In some embodiments not part of the invention, the immune checkpoint inhibitor is an inhibitor of PD-L2. In other embodiments, the immune checkpoint inhibitor reduces the interaction between PD-1 and PD-L2. Exemplary immune checkpoint inhibitors include antibodies (e.g., an anti-PD-L2 antibody), RNAi molecules (e.g., an anti-PD-L2 RNAi), antisense molecules (e.g., an anti-PD-L2 antisense RNA), dominant negative proteins (e.g., a dominant negative PD-L2 protein), and small molecule inhibitors. Antibodies include monoclonal antibodies, humanized antibodies, deimmunized antibodies, and Ig fusion proteins.

In some embodiments according to the invention, the immune checkpoint inhibitor is an inhibitor of CTLA-4. In one embodiment, the immune checkpoint inhibitor is an anti-CTLA-4 antibody. In one particular embodiment, the immune checkpoint inhibitor is ipilimumab. In one embodiment, the anti-CTLA-4 antibody blocks the binding of CTLA-4 to CD80 (B7-1) and/or CD86 (B7-2) expressed on antigen presenting cells. Exemplary antibodies against CTLA-4 include: Bristol Meyers Squibb's anti-CTLA-4 antibody ipilimumab (also known as Yervoy^{®}, MDX-010, BMS-734016 and MDX-101); anti-CTLA4 Antibody, clone 9H10 from Millipore; Pfizer's tremelimumab (CP-675,206, ticilimumab); and anti-CTLA4 antibody clone BNI3 from Abcam.

In some embodiments according to the invention, the anti-CTLA-4 antibody is, for example, disclosed in: WO 98/42752; U.S. Pat. Nos. 6,682,736 and 6,207,156; Hurwitz et al. (1998); Camacho et al. (2004) (antibody CP-675206); Mokyr et al. (1998).

In some embodiments, the CTLA-4 inhibitor is a CTLA-4 ligand as disclosed in WO1996040915. In some embodiments, the CTLA-4 inhibitor is a nucleic acid inhibitor of CTLA-4 expression.

Any suitable immune checkpoint inhibitor is contemplated for use with the compositions, dosage forms, and methods disclosed herein. The selection of the immune checkpoint inhibitor depends on multiple factors, and the selection of the immune checkpoint inhibitor is within the skills of one of skill in the art. For example, factors to be considered include any additional drug interactions of the immune checkpoint inhibitor, and the length for which the immune checkpoint inhibitor may be taken. In certain instances, the immune checkpoint inhibitor is an immune checkpoint inhibitor which may be taken long-term, for example chronically.

In embodiments where the immune checkpoint inhibitor is an antibody, the antibody may be a monoclonal antibody, synthetic antibody, polyclonal antibody, multi-specific antibody (including bi-specific antibodies), human antibody, humanized antibody, chimeric antibody, single-chain Fv (scFv) (including bi-specific scFvs), single chain antibody, Fab fragment, F(ab') fragment, disulfide-linked Fv (sdFv), and epitope-binding fragments of any of the above. In particular, antibodies for use in the present invention include immunoglobulin molecules and immunologically active portions of immunoglobulin molecules, i.e., molecules that contain a binding site for an immune checkpoint molecule that immunospecifically bind to the immune checkpoint molecule. The immunoglobulin molecules for use in the invention can be of any type (e.g., IgG, IgE, IgM, IgD, IgA and IgY), class (e.g., IgG1, IgG2, IgG3, IgG4, IgA1 and IgA2) or subclass of immunoglobulin molecule. Preferably, the antibodies for use in the invention are IgG, more preferably, IgG1.

In one embodiment, the immune checkpoint inhibitor may include without limitation humanized or fully human antibodies blocking PD-L1 such as MEDI-4736 (disclosed in WO2011066389 A1 ), MPDL328 OA (disclosed in US8217149 B2) and MIH1 (Affymetrix obtainable via eBioscience (16.5983.82)) and other PD-L1 inhibitors presently under investigation. According to this invention an immune checkpoint inhibitor is preferably selected from a CTLA-4, PD-1 or PD-L1 inhibitor, such as selected from the known CTLA-4, PD-1 or PD-L1 inhibitors mentioned above (ipilimumab, tremelimumab, labrolizumab, nivolumab, pidilizumab, AMP-244, MEDI-4736, MPDL328 OA, MIH1).

In one embodiment, the vascular disrupting agent is conjugated to the immunotherapeutic agent by a linker. In one particular embodiment, the vascular disrupting agent is conjugated to an antibody, antibody fragment or antibody mimetic, such as an affibody, a domain antibody (dAb), a nanobody, a unibody, a DARPin, an anticalin, a versabody, a duocalin, a lipocalin, or an avimer. The antibody may also serve, in addition to being an immunotherapeutic agent, a tumor targeting function. By binding to a target tumor tissue or cell where its antigen or receptor is located, the antibody directs the conjugate there. Preferably, the antigen or receptor is a tumor-associated antigen, that is, an antigen that is uniquely expressed by cancerous cells or is overexpressed by cancer cells, compared to non-cancerous cells.

Any one of several different reactive groups on the antibody can be a conjugation site, including ε-amino groups in lysine residues, pendant carbohydrate moieties, carboxylic acid groups, disulfide groups, and thiol groups. Each type of reactive group represents a trade-off, having some advantages and some disadvantages. For reviews on antibody reactive groups suitable for conjugation, see, e.g., Garnett (2001) and Dubowchik and Walker (1999). In one embodiment, the antibody is conjugated via a lysine ε-amino group. In another embodiment, the antibody is conjugated via a carbohydrate side chain, as many antibodies are glycosylated. The carbohydrate side chain can be oxidized with periodate to generate aldehyde groups, which in turn can be reacted with amines to form an imine group, such as in a semicarbazone, oxime, or hydrazone. If desired, the imine group can be converted to a more stable amine group by reduction with sodium cyanoborohydride. For additional disclosures on conjugation via carbohydrate side chains, see, e.g., Rodwell et al. (1986). In yet another embodiment, the antibody can be conjugated via a carboxylic acid group. In one embodiment, a terminal carboxylic acid group is functionalized to generate a carbohydrazide, which is then reacted with an aldehyde-bearing conjugation moiety. In yet another embodiment, the antibody can be conjugated via a disulfide group bridging a cysteine residue on the antibody and a sulfur on the other portion of the conjugate. Some antibodies lack free thiol (sulfhydryl) groups but have disulfide groups, for example in the hinge region. In such case, free thiol groups can be generated by reduction of native disulfide groups.

In yet another embodiment, the linker comprises a cleavable group. In one embodiment, the cleavable group is cleavable under physiological conditions, preferably it is relatively stable while the conjugate is in general circulation in the blood plasma, but is readily cleaved once the conjugate reaches its site of intended action, that is, near, at, or within the target tumor or tumor cell.

### Combination Treatment - Dosing and Administration

As understood in the art, the terms "combination therapy", "combination treatment", or "pharmaceutical combination" refer to the use of more than one medication or other therapy (vs. monotherapy, which is any therapy taken alone), to treat a single disease. A "Pharmaceutical combination" therapy, for example, may be achieved by prescribing/administering separate drugs, or, where available, dosage forms that contain more than one active ingredient (such as fixed-dose combinations).

The methods and uses as described herein encompass the administration of the vascular disrupting agent (combination partner a) and immunotherapeutic agent (combination partner b) to a single patient, and is intended to include treatment regimens in which the agents are not necessarily administered by the same route of administration or at the same time. Accordingly, combination partners (a) and (b) may be administered together, one after the other or separately in one combined unit dosage form or in two separate unit dosage forms. The unit dosage form may also be a fixed combination such as a pharmaceutical composition which comprises both partner (a) (or a salt, solvate or prodrug thereof) and partner (b).

In particular, a therapeutically effective amount of each of the combination partner of the combination may be administered simultaneously or sequentially and in any order, and the components may be administered separately or as a fixed combination.

For example, the method described herein but not part of the claimed invention may comprise: (i) administration of partner (a) in free or pharmaceutically acceptable salt form; and (ii) administration of partner (b) simultaneously or sequentially in any order, in jointly therapeutically effective amounts, preferably in synergistically effective amounts, e.g., in daily or intermittent dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that converts *in vivo* to the combination partner as such.

It will be appreciated that the combination partners may be presented as a "kit of parts" for use in the treatment of cancer. The kit may comprise a package where the combination partners are supplied separately for co-administration with instructions for use in the particular therapy.

The effective dosage of each of the combination partners employed in the combination of the invention may vary depending on the particular compound or pharmaceutical composition employed, the mode of administration, and the severity/grade of the cancer being treated.

Daily dosages for combination partners (a) and (b) will, of course, vary depending on a variety of factors, e.g., the compound chosen, the particular condition to be treated and the desired effect. In general, however, satisfactory results are achieved on administration of agent (a) at daily dosage rates of about 0.05 to 20 mg/kg per day, particularly 1 to 20 mg/kg per day, e.g. 0.4 to 16 mg/kg per day, as a single dose or in divided doses. Combination partner (a) and partner (b) may be administered by any conventional route, in particular enterally, e.g., orally, e.g., in the form of tablets, capsules, drink solutions or parenterally, e.g., in the form of injectable solutions or suspensions. Suitable unit dosage forms for oral administration comprise from about 0.02 to 50 mg active ingredient, usually 0.1 to 30 mg and 2 to 25 mg, 4 to 20 mg e.g. combination partner (a) or (b), together with one or more pharmaceutically acceptable diluents or carriers therefore.

The pharmaceutical combination described herein but not part of the invention may be used for the treatment of solid tumors. Examples of solid tumors include adrenocortical carcinoma, anal tumor/cancer, bladder tumor/cancer, bone tumor/cancer (such as osteosarcoma), brain tumor, breast tumor/cancer, carcinoid tumor, carcinoma, cervical tumor/cancer, colon tumor/cancer, endometrial tumor/cancer, esophageal tumor/cancer, extrahepatic bile duct tumor/cancer, Ewing family of tumors, extracranial germ cell tumor, eye tumor/cancer, gallbladder tumor/cancer, gastric tumor/cancer, germ cell tumor, gestational trophoblastic tumor, head and neck tumor/cancer, hypopharyngeal tumor/cancer, islet cell carcinoma, kidney tumor/cancer, laryngeal tumor/cancer, leiomyosarcoma, leukemia, lip and oral cavity tumor/cancer, liver tumor/cancer (such as hepatocellular carcinoma), lung tumor/cancer, lymphoma, malignant mesothelioma, Merkel cell carcinoma, mycosis fungoides, myelodysplastic syndrome, myeloproliferative disorders, nasopharyngeal tumor/cancer, neuroblastoma, oral tumor/cancer, oropharyngeal tumor/cancer, osteosarcoma, ovarian epithelial tumor/cancer, ovarian germ cell tumor, pancreatic tumor/cancer, paranasal sinus and nasal cavity tumor/cancer, parathyroid tumor/cancer, penile tumor/cancer, pituitary tumor/cancer, plasma cell neoplasm, prostate tumor/cancer, rhabdomyosarcoma, rectal tumor/cancer, renal cell tumor/cancer, transitional cell tumor/cancer of the renal pelvis and ureter, salivary gland tumor/cancer, Sezary syndrome, skin tumors (such as cutaneous t-cell lymphoma, Kaposi's sarcoma, mast cell tumor, and melanoma), small intestine tumor/cancer, soft tissue sarcoma, stomach tumor/cancer, testicular tumor/cancer, thymoma, thyroid tumor/cancer, urethral tumor/cancer, uterine tumor/cancer, vaginal tumor/cancer, vulvar tumor/cancer, and Wilms' tumor. In one embodiment, the cancer is selected from bladder cancer, breast cancer, colon cancer, gastroenterological cancer, kidney cancer, lung cancer,including non-small cell lung cancer, ovarian cancer, pancreatic cancer, prostate cancer, proximal or distal bile duct cancer, or melanoma.

### Additional Therapies

The methods described herein but not falling within the scope of the invention may utilise the combination of a vascular disrupting agent and an immunotherapeutic agent in conjunction with other therapeutic agents and treatment modalities such as tumor irradiation. For example, the combination therapy of the present invention may be used in conjunction with another chemotherapeutic, antibody and or immunotherapeutic that is suitable for administration to a patient for the treatment of cancer.

Examples of therapeutic agents that may be administered in conjunction with the combination of a vascular disrupting agent and an immunotherapeutic include tyrosine kinase inhibitors, such as VEGF-directed tyrosine kinase inhibitors and proteasome inhibitors. By way of example, tyrosine kinase inhibitors include sunitinib (Sutent), sorafenib (Nexavar), axitinib (Inlyta) and pazopanib (Votrient). Another therapeutic agent used in the treatment of renal cancer is carfilzomib (Kyprolis), a selective proteasome inhibitor. By way of nonlimiting examples, cytokines useful in the invention include interleukin 2 (IL2), and interferon alpha (IFNα).

### EXAMPLES

### Example 1. BNC105 in combination with anti-PD1

The present inventors conducted a study to determine the efficacy of BNC105 in combination with the immunotherapeutic anti-PD1 antibody in the syngeneic MC38 murine colon tumor model. BNC105P was administered at 10 mg/kg i.v. on Day 1, 8 and 15, anti-PD1 antibody (Clone RMP1-14) was administered at 3.5mg/kg i.p. on Day 1, 4, 8, 12 and 16. C57/BL6 mice were inoculated subcutaneously with MC38 cells. When tumors reached a volume of approximately 100-150mm3 animals were randomised into groups of 10 mice per group.

Groups:
1. Saline + PBS control
2. Saline + IgG2a isotype control antibody
3. BNC105P + IgG2a isotype control antibody
4. Saline + anti-PD1 antibody
5. BNC105P + anti-PD1 antibody

The in-life part of this study was completed on Day 18 of the treatment period due to tumor size in the control groups reaching the ethical limit. Tumors and blood were collected for FACS analysis and also tumors were collected for histology. FACS analysis and histology is currently under way and is aimed at identifying changes of immune T-cell populations following treatment.

Tumor growth inhibition was evident as early as Day 8 of the treatment period especially in the combination group compared to control group (p<0.05) (Figure 1A). On Day 17 of the treatment period, animals treated with BNC105 as a monotherapy experienced 40% inhibition of tumor growth, anti-PD1 treated animals experienced 74% inhibition in tumor growth. Animals treated with the combination of BNC105+anti-PD1 therapy experienced 97% inhibition in tumor growth (Figure 1B).

### Example 2. BNC105 in combination with anti-CTLA-4

The present inventors conducted a separate study to determine the efficacy of BNC105 in combination with the immune-oncology therapeutic antibody anti-CTLA4 in the syngeneic CT26 murine colon tumor model. BNC105 (10mg/kg) was administered on Days 1 and 8, the anti-CTLA4 antibody (Clone 9D9) was administered at 10mg/kg i.p. on Days 2, 5, and 9. Balb/c mice were inoculated subcutaneously with CT26 cells. When tumors reached an average volume of approximately 135mm3 animals were randomised into 5 groups of 10 mice per group.

Groups:
1. Saline + PBS control
2. Saline + IgG2b isotype control antibody
3. BNC105P + IgG2b isotype control antibody
4. Saline + anti-CTLA4 antibody
5. BNC105P + anti-CTLA4 antibody

The in-life part of this study was completed on Day 12 of the treatment period due to tumor sizes in the control groups reaching ethical limits. Tumors were collected for both FACS analysis and for histology. FACS analysis and histology are currently in progress and aim to identify changes of immune T-cell populations following treatment.

Animals treated with the BNC105+anti-CTLA4 combination experienced greater inhibition of tumor growth compared to animals treated with either BNC105 or anti-CTLA4 alone (Figure 2A). On Day 11 of the treatment period, animals treated with BNC105 as a monotherapy experienced 27% inhibition in tumor growth and anti-CTLA4 treated animals experienced 14% inhibition of tumor growth. Animals treated with the combination of BNC105+anti-CTLA4 experienced 70% inhibition in tumor growth (Figure 2B). Thus, in view of the results obtained in the experiments outlined in Examples 1 and 2, the present inventors have demonstrated a synergistic effect of combining a vascular disrupting agent and an immune checkpoint inhibitor in the treatment of cancer.

### Example 3: BNC105 disrupts immune homeostasis of tumor micro-environment

Stimulating an initial immune response by altering the immune homeostasis can be the key to making immunotherapy relevant to more patients. Accordingly, the present inventors examined key immune clinical biomarkers from BNC105 treated patients and investigate pre-clinically the potential therapeutic benefit of combining BNC105 with the checkpoint inhibitors that target PD-1 or CTLA-4.

### BNC105 causes acute tumor damage and increases tumor IFNγ

BNC105 causes rapid destruction of tumor vasculature and is highly selective leaving normal vasculature intact. In a murine orthotopic model of Renal Cancer (Renca) the present inventors have shown using perfusion of a vascular stain that tumor blood vessels are obliterated after BNC105 treatment compared to normal tissue which remains unaffected.

BNC105 primes the immunogenic potential of a tumor with increased tumoral IFNγ content compared to control treated animals (Figure 3). IFNγ is secreted from CD4+ Th1, CD8+ and Th0 cells and activated NK cells. No change in tumoral CD3+/CD8+ cells was seen suggesting an increase in the component of complementary immune cells fostering an environment for tumor specific immune activation when checkpoint inhibitors are deployed.

### BNC105 clinically enhances the immune response IL-12 p40 and IL-10

The balance between pro-inflammatory and anti-inflammatory signals provided by different immune cell populations is crucial for normal physiology and the suppression of cancer development. By altering this homeostasis an opportunity is provided for the immune system to alter the way it responds. Biomarker analysis on patient samples was conducted from a Phase II BNC105 monotherapy mesothelioma trial. Biomarker analysis showed that plasma IL-12 subunit p40 significantly increases post-BNC105 administration and remains elevated at Day 8 post dosing (Figure 4). The immune-modulatory cytokine IL-12 subunit p40, a key member of the IL-12 cytokine family, has emerged as a potent inducer of antitumor immunity. IL-12 subunit p40 is secreted by activated macrophages and serves as an essential inducer of Th1 cells development.

Significant changes were also seen in levels of the immune-modulatory cytokine IL-10 (Figure 4). IL-10 mediated stimulation of adaptive immunity to tumors has been observed clinically. IL-10 increases monocytes which are able to induce the expansion of tumor resident CD8+T cells in tumors and enhance their cytotoxic activity.

### BNC105 treatment reduces the number of infiltrating macrophages

A significant reduction in the number of tumor infiltrating macrophages (CD11b+) was seen after treatment with BNC105 (monotherapy and combination) (Figure 5). This reduction would dramatically change the immune environment potentially releasing the immune dampening effect of macrophage subsets.

The present inventors' findings strongly support a therapeutic benefit of the combination of BNC105 with immunotherapeutic agents, for example such as immune checkpoint inhibitors, and that BNC105 driven priming of the tumor and immune system should extend the reach of checkpoint inhibitors to leverage a therapeutic benefit to a greater patient population.

### references

Camacho et al. (2004) J. Clin. Oncol., 22(145): Abstract No. 2505
Dubowchik and Walker (1999) Pharmacology &Therapeutics 83, 67-123
Garnett (2001) Adv. Drug Delivery Rev. 53, 171-216
Hurwitz et al. (1998) Proc. Natl. Acad. Sci. USA, 95(17): 10067-10071
Mokyr et al. (1998) Cancer Res., 58:5301-5304
Pettit et al. (1995) Anticancer Drug Des, 10:299
Rodwell et al. (1986) Proc. Nat'l Acad. Sci. USA 83, 2632-2636

## Claims

1. A pharmaceutical combination of a vascular disrupting agent and an immunotherapeutic agent, for use in the treatment of cancer, wherein the vascular disrupting agent is a tubulin polymerisation inhibitor selected from 2-methyl-7-hydroxy-3-(3,4,5-trimethoxybenzoyl)-6-methoxybenzofuran (BNC105) and disodium[6-methoxy-2-methyl-3-(3,4,5-trimethoxybenzoyl)-1-benzofuran-7-yl] phosphate (BNC105P), and wherein the immunotherapeutic agent is an anti-immune checkpoint inhibitor antibody selected from an anti-PD-L1, an anti-PD-1 and an anti-CTLA-4 antibody.

2. An immunotherapeutic agent for use in the treatment of cancer in a cancer patient undergoing treatment with a vascular disrupting agent, wherein the vascular disrupting agent is a tubulin polymerisation inhibitor selected from 2-methyl-7-hydroxy-3-(3,4,5-trimethoxybenzoyl)-6-methoxybenzofuran (BNC105) and disodium[6-methoxy-2-methyl-3-(3,4,5-trimethoxybenzoyl)-1-benzofuran-7-yl] phosphate (BNC105P), and wherein the immunotherapeutic agent is an anti-immune checkpoint inhibitor selected from an anti-PD-L1, an anti-PD-1 and an anti-CTLA-4 antibody.

3. A vascular disrupting agent for use in the treatment of cancer in a cancer patient undergoing treatment with an immunotherapeutic agent, wherein the vascular disrupting agent is a tubulin polymerisation inhibitor selected from 2-methyl-7-hydroxy-3-(3,4,5-trimethoxybenzoyl)-6-methoxybenzofuran (BNC105) and disodium[6-methoxy-2-methyl-3-(3,4,5-trimethoxybenzoyl)-1-benzofuran-7-yl] phosphate (BNC105P), and
wherein the immunotherapeutic agent is an inhibitor anti-immune checkpoint inhibitor antibody selected from an anti-PD-1, anti-PD-L1 and anti-CTLA-4 antibody.

4. The vascular disrupting agent and/or the immunotherapeutic agent for use according to any one of claims 1 to 3, wherein the cancer is selected from bladder cancer, breast cancer, colon cancer, gastroenterological cancer, kidney cancer, lung cancer, ovarian cancer, pancreatic cancer, prostate cancer, proximal or distal bile duct cancer, melanoma.

5. The vascular disrupting agent and/or immunotherapeutic agent for use according to claim 4, wherein the cancer is colon cancer.

## Patentansprüche

1. Pharmazeutische Kombination eines vaskulären Unterbrechungsmittels und eines immuntherapeutischen Mittels zur Verwendung bei der Behandlung von Krebs, wobei das vaskuläre Unterbrechungsmittel ein Tubulinpolymerisationsinhibitor ist, ausgewählt aus 2-Methyl-7-hydroxy-3-(3,4,5-Trimethoxybenzoyl)-6-methoxybenzofuran (BNC105) und Dinatrium[6-methoxy-2-methyl-3-(3,4,5-trimethoxybenzoyl)-1-benzofuran-7-yl]phosphat (BNC105P) und wobei das immuntherapeutische Mittel ein Anti-Immun-Checkpoint-Inhibitor-Antikörper ist, ausgewählt aus einem Anti-PD-L1-, einem Anti-PD-1- und einem Anti-CTLA-4-Antikörper.

2. Immuntherapeutisches Mittel zur Verwendung bei der Behandlung von Krebs bei einem Krebspatienten, der mit einem vaskulären Unterbrechungsmittel behandelt wird, wobei das vaskuläre Unterbrechungsmittel ein Tubulinpolymerisationsinhibitor ist, ausgewählt aus 2-Methyl-7-hydroxy-3-(3,4,5-Trimethoxybenzoyl)-6-methoxybenzofuran (BNC105) und Dinatrium[6-methoxy-2-methyl-3-(3,4,5-trimethoxybenzoyl)-1-benzofuran-7-yl]-phosphat (BNC105P) und wobei das immuntherapeutische Mittel ein Anti-Immun-Checkpoint-Inhibitor ist, ausgewählt aus einem Anti-PD-L1-, einem Anti-PD-1- und einem Anti-CTLA-4-Antikörper.

3. Vaskuläres Unterbrechungsmittel zur Verwendung bei der Behandlung von Krebs bei einem Krebspatienten, der mit einem immuntherapeutischen Mittel behandelt wird, wobei das vaskuläre Unterbrechungsmittel ein Tubulinpolymerisationsinhibitor ist, ausgewählt aus 2-Methyl-7-hydroxy-3-(3,4,5-Trimethoxybenzoyl)-6-methoxybenzofuran (BNC105) und Dinatrium[6-methoxy-2-methyl-3-(3,4,5-trimethoxybenzoyl)-1-benzofuran-7-yl]phosphat (BNC105P), und
wobei das immuntherapeutische Mittel ein Anti-Immun-Checkpoint-Inhibitor-Antikörper ist, ausgewählt aus einem Anti-PD-1-, Anti-PD-L1- und Anti-CTLA-4-Antikörper.

4. Vaskuläres Unterbrechungsmittel und/oder immuntherapeutisches Mittel zur Verwendung nach einem der Ansprüche 1 bis 3, wobei der Krebs ausgewählt ist aus Blasenkrebs, Brustkrebs, Dickdarmkrebs, gastroenterologischem Krebs, Nierenkrebs, Lungenkrebs, Eierstockkrebs, Bauchspeicheldrüsenkrebs, Prostatakrebs, proximalem oder distalem Gallengangskrebs, Melanom.

5. Vaskuläres Unterbrechungsmittel und/oder immuntherapeutisches Mittel zur Verwendung nach Anspruch 4, wobei der Krebs Dickdarmkrebs ist.

## Revendications

1. Combinaison pharmaceutique d'un agent perturbateur vasculaire et d'un agent immunothérapeutique, pour une utilisation dans le traitement du cancer, dans laquelle l'agent perturbateur vasculaire est un inhibiteur de la polymérisation de la tubuline sélectionné parmi du 2-méthyl-7-hydroxy-3-(3,4,5-triméthoxybenzoyl)-6-méthoxybenzofuran (BNC105) et du disodium[6-méthoxy-2-méthyl-3-(3,4,5-triméthoxybenzoyl)-1-benzofuran-7-yl] phosphate (BNC105P), et dans laquelle l'agent immunothérapeutique est un anticorps anti-inhibiteur de point de contrôle immunitaire sélectionné parmi un anticorps anti-PD-L1, un anti-PD-1 et un anti-CTLA-4.

2. Agent immunothérapeutique pour une utilisation dans le traitement du cancer chez un patient cancéreux suivant un traitement avec un agent anti-vasculaire, dans lequel l'agent perturbateur vasculaire est un inhibiteur de la polymérisation de la tubuline sélectionné parmi du 2-méthyl-7-hydroxy-3-(3,4,5-triméthoxybenzoyl)-6-méthoxybenzofuran (BNC105) et du disodium[6-méthoxy-2-méthyl-3-(3,4,5-triméthoxybenzoyl)-1-benzofuran-7-yl] phosphate (BNC105P), et dans lequel l'agent immunothérapeutique est un anti-inhibiteur de point de contrôle immunitaire sélectionné parmi un anticorps anti-PD-L1, un anti-PD-1 et un anti-CTLA-4.

3. Agent perturbateur vasculaire pour une utilisation dans le traitement du cancer chez un patient cancéreux suivant un traitement avec un agent immunothérapeutique, dans lequel l'agent perturbateur vasculaire est un inhibiteur de la polymérisation de la tubuline sélectionné parmi du 2-méthyl-7-hydroxy-3-(3,4,5-triméthoxybenzoyl)-6-méthoxybenzofuran (BNC105) et du disodium[6-méthoxy-2-méthyl-3-(3,4,5-triméthoxybenzoyl)-1-benzofuran-7-yl] phosphate (BNC105P), et
dans lequel l'agent immunothérapeutique est un anticorps anti-inhibiteur de point de contrôle immunitaire sélectionné parmi un anticorps anti-PD-1, un anti-PD-L1 et un anti-CTLA-4.

4. Agent perturbateur vasculaire et/ou agent immunothérapeutique pour une utilisation selon l'une quelconque des revendications 1 à 3, dans lequel le cancer est sélectionné parmi un cancer de la vessie, un cancer du sein, un cancer du côlon, un cancer de l'appareil digestif, un cancer du rein, un cancer du poumon, un cancer de l'ovaire, un cancer du pancréas, un cancer de la prostate, un cancer des voies biliaires proximales ou distales, un mélanome.

5. Agent perturbateur vasculaire et/ou agent immunothérapeutique pour une utilisation selon la revendication 4, dans lequel le cancer est un cancer du côlon.
